# EUROPEAN PATENT APPLICATION

(11) **EP 4 035 673 A1**
(43) Date of publication of application: **03.08.2022**
(21) Application number: 21386013.3
(22) Date of filing: 01.02.2021
(51) Int. Cl.: A61K 39/00, A61K 39/12, A61P 31/14

(54) **TRANSDERMAL VACCINE**

(71) Applicant: Oxford University Innovation Limited, Oxford, OX2 0JB (GB)
(72) Inventor: The designation of the inventor has not yet been filed
(74) Representative: J A Kemp LLP

(57) **Abstract**

The invention describes transdermal vaccines which contain ultrasound responsive particles comprising a polypeptide shell. The surface of the particle has one or more indentations which are generally able to entrap a gas bubble. The particles are capable of generating inertial cavitation on exposure to ultrasound. The particles can be delivered transdermally, and can comprise antigen protein and/or adjuvant within the particle structure. The particles are therefore useful in methods of vaccination using transdermal delivery routes.

## Description

### FIELD OF THE INVENTION

The present invention relates to a composition comprising ultrasound-responsive polypeptide particles. The particles can be delivered transdermally, by use of ultrasound-induced inertial cavitation, and are useful as transdermal vaccine compositions or carriers for transdermally administered vaccines. The compositions can be used in methods of vaccination.

### BACKGROUND

Vaccines have an important role to play in today's society, in particular given the continued threat of pandemic and the need for long term vaccination strategies for known infectious diseases such as influenza. Although many vaccines are administered by injection, this delivery route is associated with disadvantages such as pain, needle stick injuries, phobia and poor compliance. Transdermal vaccines are therefore an attractive option which might address these issues.

However, the delivery of vaccines transdermally presents a number of difficulties. Techniques which have been trialled include use of ultrasound to permeablise the skin, creating pores through which subsequently added vaccine molecules can pass. However, this has not led to sufficiently effective delivery.

Microneedle-assisted delivery is a further technique which has been studied. Microneedles create pores in the skin which are designed to enable vaccine molecules to pass through the skin via the pores created. Transdermal delivery using microneedles has been used to administer polio vaccine in volunteers and to investigate bacillus Calmette-Guerin (BCG) vaccine in animal models. However, it is not suitable for the delivery of high doses of vaccine and can be associated with low patient compliance.

Despite the significant amount of development in transdermal vaccination, there has been no breakthrough technology which provides a simple and effective technique capable of competing with the current widespread use of injection. New transdermal vaccination technologies are therefore needed.

### SUMMARY OF THE INVENTION

The present inventors have developed biodegradable, ultrasound-responsive particles made from polypeptides (e.g. proteins). The particles generate inertial cavitation on response to ultrasound and this property provides the ability to administer both the particles, and any co-administered substances, transdermally. Application of the particles of the invention to the skin, followed by applying ultrasound, generates an inertial cavitation effect sufficient to drive the particles through the skin and provide transdermal administration. The use of ultrasound may additionally provide a sonoporation effect (enlarging pores in the skin), but the movement of particles across the skin (sonophoresis) is significantly enhanced by the shock wave and/or microstreaming effects generated by the inertial cavitation.

Ultrasound-responsive particles have been used for some time to assist delivery of therapeutic or diagnostic agents to the body. For instance, drug can be co-administered with microbubbles, which act as cavitation nuclei. The action of ultrasound on the microbubbles leads to a short burst of microstreaming caused by the cavitation effects. This can increase the delivery of the drug at the target site.

However, microbubbles provide only a very short duration of action, with cavitation lasting for no more than about 30 seconds. They are also unsuitable for transdermal administration.

Nanoparticulate cavitation agents have also been described (WO 2015/075422) which have the benefit of longer duration of action and have a smaller size which provides greater utility. The particles described are formed by seed polymerisation to generate cup-shaped plastic particles, capable of holding a gas bubble in the cavity of the cup. These particles have found use as injectable cavitation agents. However, the particles described are plastic and are non-biodegradable. Moreover, there has been no suggestion of the use of such particles in transdermal administration.

The particles described herein can be produced at submicron sizes suitable for transdermal administration, and have a good duration of action, having been found to generate cavitation for a period of around 10 minutes following ultrasound exposure. The particles are highly stable, but are also biodegradeable. The particles therefore provide a number of advantages compared with known ultrasound-responsive particles and, in particular, they have demonstrated effective transdermal delivery.

Not only can the particles described herein be delivered transdermally, they can also themselves act as vaccine and/or adjuvant, and may therefore be useful as a transdermally administered vaccine composition. The present invention therefore provides a first aspect (1):
1. A transdermal vaccine composition comprising a plurality of polypeptide particles and at least one pharmaceutically acceptable carrier or diluent, wherein the plurality of polypeptide particles are suitable for inducing cavitation on exposure to ultrasound, and wherein each particle comprises:
   - a polypeptide shell; and optionally
   - a core comprising a water immiscible liquid and/or one or more adjuvants;
   wherein the particle has one or more surface indentations.

The particles of the transdermal vaccine composition may comprise a pathogenic antigen protein in the polypeptide shell. It has surprisingly been found that a significant immune response is generated to proteins present in the polypeptide shell, suggesting a high level of efficacy when used as a vaccine. Thus, particles according to the invention having an antigen protein in the shell, are able to generate an immune response to the antigen protein. Moreover, the core of the particle can conveniently comprise an adjuvant, boosting the immune response achieved. Alternatively, particles according to the invention may comprise an adjuvant polypeptide in the shell, and be administered together with a vaccine. The particles are therefore useful as transdermally administered vaccines, or carriers for transdermally administered vaccines such as adjuvant compositions.

The present invention also provides the following aspects:
2. A transdermal vaccine composition according to aspect 1, wherein the polypeptide shell comprises a pathogenic antigen protein and/or wherein the composition further comprises a vaccine, preferably a DNA or RNA vaccine.
3. A transdermal vaccine composition according to aspect 1 or aspect 2, wherein the core comprises one or more adjuvants.
4. A transdermal vaccine composition according to aspect 3, wherein the one or more adjuvants are selected from imiquimod and squalene.
5. A transdermal vaccine composition according to any one of the preceding aspects, wherein the polypeptide shell comprises a pathogenic antigen protein.
6. A transdermal vaccine composition according to aspect 5, wherein the pathogenic antigen protein is selected from, a covid spike protein, HepB surface antigen protein, hemagglutinin, an influenza neuraminidase, filamentous hemagglutinin, pneumococcal surface protein A, Neisserial adhesin A, Neisserial Heparin Binding Antigen, factor H binding protein, and HPV-16 E6 or E7 fusion protein.
7. A transdermal vaccine composition according to any one of aspects 1 to 6, wherein the polypeptide shell comprises an adjuvant polypeptide.
8. A transdermal vaccine composition according to aspect 7, wherein the adjuvant polypeptide is one or more polypeptides selected from non-human albumin proteins, sFLT ligand, cytokines and chemokines, macrophage colony stimulating factor (M-CSF), tumour necrosis factor (TNF), and granulocyte and macrophage colony stimulating factor (GM-CSF).
9. A transdermal vaccine composition according to any one of aspects 1 to 8 wherein the composition further comprises a DNA or RNA vaccine.
10. A transdermal vaccine composition according to any one of the preceding aspects, wherein the particle size is in the range of from 100nm to 10000nm, preferably from 200nm to 1000nm.
11. A transdermal vaccine composition according to any one of aspects 1 to 9, wherein the particle size is more than 600nm, preferably more than 700nm, preferably in the range of between 600nm and 10000nm, preferably between 700nm and 10000nm, more preferably between 700nm and 1000nm.
12. A transdermal vaccine composition according to any one of aspects 1 to 9, wherein the particle size is more than 1000nm, preferably in the range of between 1000nm and 10000nm, preferably between 1000nm and 5000nm.
13. A transdermal vaccine composition according to any one of the preceding aspects, wherein the cross section of at least one surface indentation forms a conic section.
14. A transdermal vaccine composition according to any one of the preceding aspects, wherein at least one surface indentation has a depth of at least 10nm, and/or an opening size of at least 50nm.
15. A transdermal vaccine composition according to any one of the preceding aspects, wherein the particle comprises one or two indentations having an opening size which is 20% or more of the size of the particle.
16. A transdermal vaccine composition according to any one of the preceding aspects, wherein the polypeptide shell is conjugated to one or more ligands selected from a peptide, a protein, a sugar, a nanoparticle and a nucleic acid.
17. A transdermal vaccine composition according to any one of the preceding aspects, which particle is capable of generating inertial cavitation when the particle is suspended in water and subject to ultrasound at 1.8MPa and 265kHz.
18. A polypeptide particle for inducing cavitation on exposure to ultrasound, the particle comprising:
   - a polypeptide shell comprising a pathogenic antigen protein as defined in aspect 5 or aspect 6; and optionally
   - a core comprising a water immiscible liquid and/or one or more adjuvants, the adjuvants being as defined in aspect 3 or aspect 4;
   wherein the particle has one or more surface indentations.
19. A polypeptide particle for inducing cavitation on exposure to ultrasound, the particle comprising:
   - a polypeptide shell wherein the polypeptide shell optionally comprises a pathogenic antigen protein as defined in aspect 5 or aspect 6; and
   - a core comprising one or more adjuvants and optionally a water-immiscible liquid, wherein the adjuvants are as defined in aspect 3 or aspect 4;
   wherein the particle has one or more surface indentations.
20. A polypeptide particle for inducing cavitation on exposure to ultrasound, the particle comprising:
   - a polypeptide shell comprising an adjuvant polypeptide as defined in aspect 7 or aspect 8; and optionally
   - a core comprising a water immiscible liquid and/or one or more adjuvants, the adjuvants being as defined in aspect 3 or aspect 4;
   wherein the particle has one or more surface indentations, and preferably wherein the particle has a particle size of more than 700nm.
21. A polypeptide particle according to aspect 18 or 19, wherein the particle size is in the range of from 100nm to 10000nm, preferably from 200nm to 1000nm.
22. A pharmaceutical adjuvant composition comprising a plurality of polypeptide particles according to aspect 19 or aspect 20 together with one or more pharmaceutically acceptable carriers or diluents.
23. A polypeptide particle according to any one of aspects 18 to 20, or a composition according to aspect 22, wherein the polypeptide particle is as defined in any one of aspects 11 to 17.
24. A method of producing a polypeptide particle according to aspect 18, comprising:
   - providing a water-immiscible phase comprising one or more volatile components, optionally one or more non-volatile components, and optionally one or more adjuvants as defined in aspect 3 or aspect 4;
   - mixing said water-immiscible phase with an aqueous solution of at least one polypeptide, wherein the at least one polypeptide comprises a pathogenic antigen protein as defined in aspect 5 or aspect 6;
   - cross-linking the polypeptide to generate a particle having a core comprising the water-immiscible phase and a shell comprising at least one polypeptide; and
   - creating one or more indentations in the particle by subjecting the particle to reduced pressure conditions, to remove volatile component from the core.
25. A method of producing a polypeptide particle according to aspect 19, comprising:
   - providing a water-immiscible phase comprising one or more volatile components, one or more adjuvants as defined in aspect 3 or aspect 4, and optionally one or more non-volatile components;
   - mixing said water-immiscible phase with an aqueous solution of at least one polypeptide, wherein the at least one polypeptide optionally comprises a pathogenic antigen protein as defined in aspect 5 or aspect 6;
   - cross-linking the protein to generate a particle having a core comprising the water-immiscible phase and a shell comprising the at least one polypeptide; and
   - creating one or more indentations in the particle by subjecting the particle to reduced pressure conditions, to remove volatile component from the core.
26. A method of producing a polypeptide particle according to aspect 20, comprising:
   - providing a water-immiscible phase comprising one or more volatile components, optionally one or more non-volatile components, and optionally one or more adjuvants as defined in aspect 3 or aspect 4;
   - mixing said water-immiscible phase with an aqueous solution of at least one polypeptide, wherein the at least one polypeptide comprises an adjuvant polypeptide as defined in aspect 7 or aspect 8;
   - cross-linking the polypeptide to generate a particle having a core comprising the water-immiscible phase and a shell comprising at least one polypeptide; and
   - creating one or more indentations in the particle by subjecting the particle to reduced pressure conditions, to remove volatile component from the core.
27. A method according to any one of aspects 24 to 26, wherein the ratio of volatile component to non-volatile component in the water-immiscible phase is from 1:20 to 20:1.
28. A method according to any one of aspects 24 to 27, which further comprises:
   - drying the particle; and
   - optionally re-suspending the particle in a liquid medium.
29. A method according to any one of aspects 24 to 28, which further comprises:
   - lyophilising the particle; and
   - optionally re-suspending the particle in a liquid medium;
   wherein preferably the lyophilisation and optional resuspension are carried out after drying the particle and re-suspending the particle in a liquid medium.
30. A method according to any one of aspects 24 to 29, which further comprises a step of purification of the particles.
31. A polypeptide particle or composition according to any one of aspects 1 to 23, for use in a method of vaccination.
32. A polypeptide particle or composition for use according to aspect 31, wherein the method comprises applying the polypeptide particle or composition to the skin, applying ultrasound and inducing inertial cavitation, such that the polypeptide particle or composition is delivered transdermally.
33. A polypeptide particle or composition for use according to aspect 31 or 32, wherein ultrasound is delivered at a frequency of from 100 to 500 kHz and a peak negative pressure of from 0.5 MPa to 2.0 MPa.
34. A polypeptide particle or composition for use according to any one of aspects 31 to 33, wherein the polypeptide particle or transdermal vaccine composition is administered together with a DNA or RNA vaccine.
35. Use of a polypeptide particle or composition according to any one of aspects 1 to 23, in the manufacture of a medicament for use in a method of vaccination, in particular wherein the method is as defined in any one of aspects 32 to 34.
36. A method of vaccination of a subject, which method comprises administering to a subject a prophylactically effective amount of a polypeptide particle or composition according to any one of aspects 1 to 23, in particular wherein the method is as described in any one of aspects 32 to 34.

### BRIEF DESCRIPTION OF THE FIGURES

Figure 1 is a schematic representation of a particle according to the invention having a single indentation. S represents the particle size and d represents the depth of the indentation. P represents the size of the opening of the indentation, also referred to as opening diameter.
Figure 2 provides a schematic representation of the process used to make the ultrasound-responsive particles of the invention, as well as a process for making substantially spherical particles.
Figures 3a and b show 3D reconstruction of Z-stacks generated via spinning disc confocal microscopy with particles pre-conjugated with AlexaFluor 488 NHS (Fig 3a) and CY5 NHS (Fig 3b); Figure 3c shows scanning electron microscopy (SEM) images of particles according to the invention; Figure 3d shows a light microscopy image of a particle according to the invention. Figure 3e(I) shows the quantity of imiquimod plotted against corresponding fluorescence peak area detected (Ex260nm Em340nm); Figure 3e(II) shows the UV absorbance profile of imiquimod.
Figure 4 shows the average diameter (Z-average) of particles generated by varying the nature of the volatile component inside the core.
Figure 5 shows the average diameter (Z-average) of particles generated by varying the relative amount of the volatile component inside the core.
Figure 6 shows the effect of particle size on cavitation ability across four pressure settings.
Figure 7 shows the effect of percentage volatile component in core on cavitation ability across four pressure settings.
Figure 8 shows a representative energy trace demonstrating persistent inertial cavitation of particles produced according to the invention.
Figure 8A shows the energy detected by PCD over time at 1.7 MPa during in vivo transdermal delivery of particles of the invention and luciferase pDNA.
Figure 9 provides a schematic of the ultrasound hardware used in in vivo cavitation experiments
Figure 10 shows: (a) Day-7 ELISA comparing the immune response to BSA protein, following delivery of particles made from BSA both transdermally and via a range of injections; (b) Avidity ELISA to quantify the binding affinity of antibodies generated.
Figure 11 provides a graph of bioluminescence as quantified via IVIS imaging, showing the extent of delivery of DNA encoding luciferase via various modes of administration, including transdermal administration of DNA together with particles as described herein.
Figure 12 shows results of an ELISA quantifying the binding of native Cetuximab protein and protein particles generated from Cetuximab to EGFR (native receptor).
Figure 13 shows the results of an ELISA specific to covid spike protein taken 21 days after administration of covid spike protein via various modes of administration.

### DETAILED DESCRIPTION OF THE INVENTION

### Definitions

As used herein, an ultrasound-responsive particle is a particle that produces a response, typically a cavitation response, when exposed to ultrasound. The particles of the invention which are suitable for inducing (i.e. capable of inducing, or adapted to induce) cavitation on exposure to ultrasound are ultrasound-responsive particles as described herein.

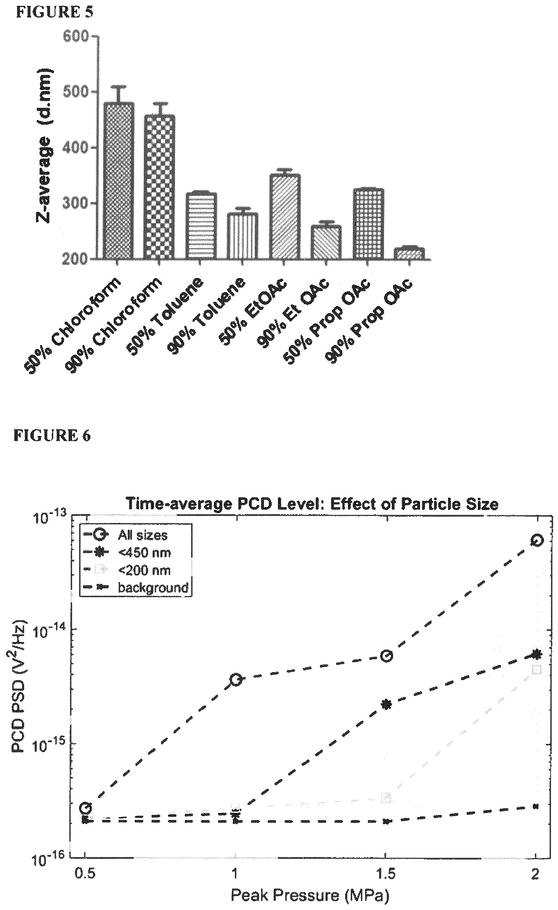

Cavitation occurs when a bubble in a fluid, e.g. a bubble associated with a particle of the invention, changes size or shape due to an external input, typically acoustic pressure. Typically, the particle generates an inertial cavitation response when exposed to ultrasound. Inertial cavitation is the effect which occurs when a bubble in a fluid, e.g. a bubble associated with a particle of the invention, grows following exposure to the acoustic pressure, and subsequently collapses. The bubble collapse may cause a shock wave and/or microstreaming in the surrounding fluid, and is accompanied by broadband (as opposed to tonal) acoustic emissions. Inertial cavitation may be detected by single- or multi-element detectors, including conventional hand held ultrasound arrays used for common medical diagnostics. Inertial cavitation may be assessed as the presence of broadband noise at a level clearly discernible from the background (e.g. at least 3 times the background root-mean-square noise) after excluding any tonal components. Particles are considered to generate an inertial cavitation response if broadband noise (e.g. measured as described above) is present on application of ultrasound (e.g. at 1.8MPa and 265kHz) to a suspension of particles in water. Inertial cavitation is demonstrated, for example as shown in Figure 6 for particles having a range of sizes, by detection of passive cavitation (PCD) levels.

Particle size as defined herein is the maximum distance across a particle, i.e. the diameter in the case of a spherical particle. Although the particles defined herein are not necessarily spherical in shape, the particle size may be referred to herein as a particle diameter and both terms have the same meaning.

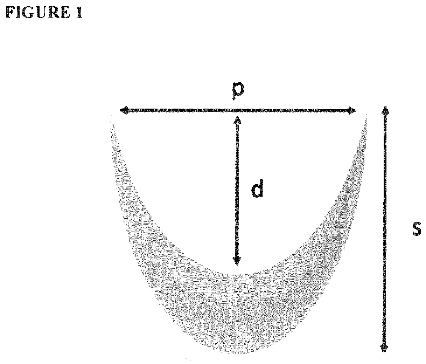

Particle size in the case of a composition containing a plurality of particles according to the invention is defined as the mean particle size. Mean particle size within a composition may be measured by a variety of techniques known to the skilled person (Malvern Nanosight, Beckman Coulter particle size, dynamic light scattering (DLS)). Typically the mean particle size is defined as the hydrodynamic diameter, as measured by dynamic light scattering (DLS). Particle size of an individual nanoparticle (s in Figure 1) may be measured by scanning electron microscopy (SEM).

As used herein, an indentation is a surface feature causing a cavity or depression to arise in the surface of the particle. Indentations are also referred to herein as surface indentations. Typically, an indentation is capable of entrapping or holding a gas bubble. The indentation has a depth depicted as d in Figure 1, which can be determined by microscopy (e.g. SEM or transmission microscopy, TEM). The depth of any indentation is typically at least about 10nm, preferably at least about 20nm, more preferably at least about 50nm. An indentation has an opening, which in the case of a part spherical indentation is the diameter of the indentation at the surface of the particle. The opening is depicted as p in Figure 1. The size of the opening is referred to herein as the opening size or the opening diameter, although the opening may or may not be circular in shape.

The shell thickness is the average thickness of the polypeptide shell and can be determined by SEM or TEM.

As used herein, the term "polypeptide" refers to a full-length protein, a portion of a protein, or a peptide characterized as a string of amino acids. Typically a polypeptide comprises at least 25, or 30, or 35, or 40, or 45, or 50 or 55 or 60 amino acids. For example, a polypeptide may contain 100 or more amino acids. As used herein, the term "protein" refers to a full length protein or a fragment of a protein.

The terms "fragment", "fragment of a protein" or "fragment of a polypeptide" as used herein refer to a string of amino acids or an amino acid sequence typically of reduced length relative to the or a reference protein or polypeptide and comprising, over the common portion, an amino acid sequence identical to the reference protein or polypeptide. In some cases the fragments referred to herein may be between 8 or 9 and 20, or 25, or 30, or 35, or 40, or 45, or 50 amino acids.

The term volatile as used herein indicates that a substance has a vapour pressure at 25°C greater than that of water (which has a vapour pressure about 3kPa) at the same temperature. Typically, a volatile substance, or a volatile component, is capable of vaporising at room temperature (25°C). A volatile component as used herein is thus a liquid, typically an organic solvent, which has a vapour pressure of at least 3 kPa, preferably at least 3.5 kPa, for example at least 5 kPa, at least 10 kPa or at least 15 kPa at 25°C.

Non-volatile indicates that a substance does not or substantially does not vaporise at room temperature (25°C). Non-volatile components are thus typically liquids, e.g. oils, which have a vapour pressure at 25°C which is no more than that of water (which has a vapour pressure about 3kPa) at the same temperature. A non-volatile component as used herein typically has a vapour pressure at 25°C of less than 3 kPa, preferably less than 2.5kPa, e.g. less than 2 kPa.

As described herein, the particles are useful in methods of vaccination of a subject. The subject is a human or animal subject. In one aspect, the subject to be treated is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters.

The term "vaccination" as used herein refers to a prophylactic treatment. Administration is typically in a "prophylactically effective amount", this being sufficient to result in a clinical response or to show clinical benefit to the individual, e.g. an effective amount to prevent or delay onset of a disease or condition.

The term "transdermal vaccine composition" as used herein refers to a composition which is suitable for transdermal administration and which can be used for the vaccination of a subject. A transdermal vaccine composition contains a vaccine, i.e. an active agent suitable for inducing an immune response in a subject. A vaccine may, for example, be a DNA or RNA vaccine or an antigen for inducing an immune response to a pathogen. For example, a transdermal vaccine composition may comprise a DNA or RNA vaccine and/or a pathogenic antigen protein. The vaccine may be a part of the polypeptide shell of the particle itself, a ligand attached to the polypeptide shell of the particle, and/or may be provided as a separate component in the composition. Transdermal vaccine compositions may additionally comprise an adjuvant.

The term "adjuvant" as used herein refers to a substance which is used to enhance immunomodulation. Adjuvants include both small molecule and protein-based substances which act to boost the immune response, e.g. to a vaccine. Immuno-stimulators such as cytokines and chemokines are examples of adjuvants as used herein. The term "adjuvant polypeptide" as used herein is a polypeptide capable of acting as an adjuvant. The term "adjuvant composition" as used herein is a composition comprising an adjuvant and which may optionally also contain a vaccine.

An immunomodulatory polypeptide (or immunomodulatory protein) is a polypeptide (or protein) which induces an immune response on administration to a subject. Typically, the subject is a human and the protein is one which induces an immune response on administration to a human subject. Typically, administration of an immunomodulatory polypeptide (or immunomodulatory protein) causes the subject to raise antibodies against the polypeptide (or protein), although alternative immune responses may arise as well as or instead of raising antibodies.

A non-immunomodulatory polypeptide (or non-immunomodulatory protein) is a polypeptide (or protein) which does not give rise to an immune response on administration. Typically, such a polypeptide or protein is one which occurs naturally within the subject. An example of a non-immunomodulatory protein in the case of a human subject is human serum albumin. More generally, human blood proteins are examples of non-immunomodulatory proteins, including insulin, globulin and haemoglobin.

### Ultrasound-responsive polypeptide particles

The polypeptide particles of the invention are ultrasound-responsive particles. The particles are capable of generating a cavitation response when exposed to ultrasound in the presence of a fluid. Typically they generate inertial cavitation. Therefore, when the particles of the invention are present in a fluid, for example formulated into a gel or lotion, or present within the body, exposure to ultrasound will lead to inertial cavitation within that fluid.

The particles have one or more indentations (i.e. surface depressions, or cavities), which provides their ultrasound-responsive properties. Without being limiting on the invention, it is currently understood that the indentations or cavities in the particle surface are able to trap or hold a gas bubble. On exposure to ultrasound, the gas bubble grows and then collapses, causing an inertial cavitation effect. Thus, the particles have one or more indentations capable of causing inertial cavitation on exposure to ultrasound. In one aspect, the invention provides particles as described herein comprising a gas bubble entrapped at an indentation on the particle. The gas bubble may comprise air or oxygen, for example.

Typically, the particles are capable of causing inertial cavitation when suspended in water and subject to ultrasound at 1.8MPa and 265kHz, e.g. at 1.2MPa and 265kHz. As described above, the ability of a particle to cause inertial cavitation can be assessed as the presence of broadband noise in the emitted signal. Preferably, the particles are capable of generating inertial cavitation when the particle is suspended in water and subject to ultrasound at 1MPa and 500kHz.

The particles typically have an average size of 100nm to 10000nm, preferably they are nanoparticles having a particle size of 1000nm or less. Preferably, the particles have a size of from 100nm to 8000nm, preferably from 100nm to 5000nm, more preferably from 100nm to 1000nm . For example, the particles preferably have a size of from 200nm to 1000nm, for example from 200nm to 700nm, for example around 500nm. Preferably, the mean particle size of a composition comprising one or more particles of the invention is from 100nm to 10000nm, preferably from 100nm to 8000nm, preferably from 100nm to 5000nm, preferably from 100nm to 1000nm, more preferably from 200nm to 1000nm, for example from 200nm to 700nm, for example around 500nm. Particle size can be controlled, for example, by size filtration of particles.

In one aspect, the particles have a particle size of more than 500nm, preferably more than 600nm, for example from 600nm to 10000nm, e.g. from 600nm to 8000nm, preferably from 600nm to 5000nm or from 700nm to 1000nm. In the case of a composition, the mean particle size may be more than 500nm, preferably more than 600nm, for example from 600nm to 10000nm, e.g. from 600nm to 8000nm, preferably from 600nm to 5000nm or from 700nm to 1000nm.

In one embodiment, particles comprising an adjuvant polypeptide in the polypeptide shell have a particle size of more than 600nm, for example from 600nm to 10000nm, e.g. from 600nm to 8000nm, preferably from 600nm to 5000nm or from 700nm to 1000nm. In the case of a composition, the mean particle size may be more than 500nm, preferably more than 600nm, for example from 600nm to 10000nm, e.g. from 600nm to 8000nm, preferably from 600nm to 5000nm or from 700nm to 1000nm.

Typically, the particles comprise a single indentation or comprise two indentations (in the latter case they are typically approximately toroid shaped, comprising two indentations, on opposite sides of the particle). Further smaller particle features, e.g. surface roughness wherein features have an opening size of 50nm or less, preferably 20nm or less, more preferably 10nm or less, may also be present. Typically, however, the particle has one or two indentations having an opening size of 50nm or greater.

The form of the indentation(s) may vary, as long as the indented particle is capable of causing inertial cavitation (e.g. the indentation(s) are capable of entrapping or holding a gas bubble within their cavity). Typically, an indentation capable of causing inertial cavitation has an opening size of at least 50nm or at least 100nm. The opening is, for example from 10 to 500nm, e.g. from 50 to 300nm or from 100 to 300nm. The opening size of an indentation as referred to herein relates to the diameter of the opening at the surface of the particle, in the case of a circular opening. In the case of a cavity having a non-circular cross-section at the opening, the opening size is the maximum dimension across the opening at the surface of the particle.

The size of the opening of an indentation may vary dependent on the size of the particle. For instance, the opening of an indentation may have a size (a diameter) which is at least 10% of the size (diameter) of the particle, e.g. at least 20% of the size (diameter) of the particle, preferably at least 40% of the size (diameter) of the particle. The opening size (diameter) of an indentation or cavity in the particle (its absolute size or relative size compared to the particle size) may be determined by imaging, e.g. SEM or AFM.

Typically, an indentation capable of causing inertial cavitation has a depth of at least 10nm, e.g. at least 50nm or at least 100nm. For example, the one or more indentations may have a depth of from 10nm to 250nm. The depth of the one or more indentations may be, for example, at least 10% of the particle size, for example at least 20% or at least 25% of the particle size. The depth of an indentation or cavity in the particle (its absolute size or relative size compared to the particle size) may be determined by imaging, e.g. SEM or AFM.

Typically, the cross-section of at least one indentation forms a conic section.

In one embodiment, the particle has a core comprising one or a mixture of non-volatile, water-immiscible components (hereinafter a "water-immiscible core"). The content of non-volatile component (also referred to as a water-immiscible liquids) as a proportion of the particle may vary widely. For example, the particle may comprise from 0.1 to 70% by weight non-volatile component and from 30 to 99.9% by weight polypeptide shell The non-volatile component is typically an oil. The nature of the non-volatile component is not particularly limited and any biocompatible, pharmaceutically acceptable oil or mixture thereof can be used. Suitable components include sunflower oil, olive oil, soybean oil, coconut oil, safflower oil, cotton seed oil, sesame oil, orange oil, limonene oil, polyethylene glycols, or other non-volatile organic solvents and combinations of two or more of these oils. Sunflower oil, olive oil or a combination of these oils is preferred. The biocompatible, pharmaceutically acceptable oil is typically not itself pharmaceutically active.

In one aspect, the water-immiscible core consists essentially of, or consists of, one or more non-volatile components. For example, in one embodiment, the water-immiscible core consists essentially of, or consists of, one or a mixture of pharmaceutically acceptable excipients which are biocompatible, pharmaceutically acceptable oils, typically the biocompatible, pharmaceutically acceptable oils listed above. In a preferred aspect of this embodiment, the water-immiscible core consists essentially of, preferably consists of sunflower oil, olive oil or a combination of these oils. In this context, the expression "consists essentially of' means that the water-immiscible core typically contains no more than 5% by weight (e.g. no more than 2%, or no more than 1% by weight, preferably no more than 0.5% by weight, more preferably no more than 0.1 % or 0.01% by weight) of other substances, e.g. substances other than non-volatile components, with reference to the total weight of the core. Thus, the water-immiscible core may comprise at least 95% by weight, preferably at least 98%, 99% by weight, more preferably at least 99.5 or 99.9% and most preferably at least 99.99% by weight of one or more pharmaceutically acceptable excipients which are biocompatible, pharmaceutically acceptable oils. The biocompatible, pharmaceutically acceptable oils are typically non-pharmaceutically active, and are preferably selected from those oils set out above.

In one embodiment, the core of the particle comprises one or more adjuvants. The one or more adjuvants may, for example, be one or more selected from adjuvants soluble in an oilbased solvent. Suitable examples include aluminum salts such as alum, aluminum hydroxide or aluminum phosphate, calcium salts (e.g. calcium phosphate hydroxide), iron salts, zinc salts, an insoluble suspension of acylated tyrosine, acylated sugars, cationically or anionically derivatised saccharides, polyphosphazenes, biodegradable microspheres, monophosphoryl lipid A (MPL), lipopolysaccharides, lipid A derivatives (e.g. of reduced toxicity), 3-O-deacylated MPL [3D-MPL], detergents, e.g. quil A, Saponin, QS21, Freund's Complete Adjuvant, Freund's Incomplete Adjuvant (Difco Laboratories, Detroit, Mich.), Merck Adjuvant 65 (Merck and Company, Inc., Rahway, N.J.), AS-2 (Smith-Kline Beecham, Philadelphia, Pa.), CpG oligonucleotides, oils, e.g. squalene, mineral oils e.g. paraffin oil, food-based oils e.g. adjuvant 65 (derived from peanut oil), bacterial products, e.g. killed bacteria selected from *Bordatella pertussis, Mycobacterium bovis,* toxoids, bioadhesives and mucoadhesives, microparticles, liposomes, polyoxyethylene ether formulations, polyoxyethylene ester formulations, muramyl peptides or imidazoquinolone compounds (e.g. imiquamod and its homologues). Human immunomodulators suitable for use as adjuvants include cytokines such as interleukins (e.g. IL-1, IL-2, IL-4, IL-5, IL-6, IL-7, IL-12, etc), macrophage colony stimulating factor (M-CSF), tumour necrosis factor (TNF), granulocyte and macrophage colony stimulating factor (GM-CSF). These immunomodulators are further examples of adjuvants which may be incorporated in the core. In a particular embodiment, the core comprises an imidazoquinolone compound, e.g. imiquimod. In one embodiment, the adjuvant is imiquimod or squalene or a mixture thereof.

In one embodiment, the core of the particle does not comprise water-immiscible liquid(s), or water-immiscible liquid is present only in a small amount, for example in an amount sufficient to stabilise or solubilise any adjuvant. Typically, in this embodiment, the water-immiscible liquid is present in an amount of no more than 10 wt%, e.g. no more than 5 wt% compared to the total weight of the particle. In this embodiment, the core of the particle may comprise one or more adjuvants, which may be present in the core in either solid or liquid form, or they may be dissolved in, or mixed with, a small amount of a water-immiscible liquid (e.g. up to 10 wt%, or up to 5 wt% water-immiscible liquid compared to the total weight of the particle). One or more pharmaceutically acceptable carriers or excipients may also be provided in the core.

The particles of this embodiment, having no, or a small amount of water-immiscible liquid, have been found to be particularly responsive to ultrasound. Thus, they effectively generate inertial cavitation when exposed to ultrasound.

Where the core of the particle does not comprise water-immiscible liquid(s) or adjuvant, the core of the particle may be hollow. Alternatively the polypeptide shell may extend to the core of the particle (in other words, the polypeptide shell also forms the core of the particle; or the particle may be considered as having no separate core). In this embodiment, reference to the "core" of the particle indicates the central part of the particle, irrespective of whether that central part is hollow or is formed by the polypeptide shell extending to the centre of the particle.

In one embodiment, the particle substantially consists of, or consists of, polypeptide (or it substantially consists of the polypeptide shell as described herein). "Substantially consists of" in this context indicates that the particle consists of at least 95% by weight, preferably at least 98%, 99% by weight, more preferably at least 99.5 or 99.9% and most preferably at least 99.99% by weight of polypeptide (or the polypeptide shell as described herein).

In one embodiment, the core of the particle does not contain any diagnostic or therapeutic component. In one embodiment, the core of the particle does not contain an adjuvant. Where the core does not contain a therapeutic or diagnostic component and/or an adjuvant, typically, such component is present in no more than a trace amount, i.e. it is present in an amount which is not sufficient to have any pharmaceutical effect. Typically, such component is present in an amount of no more than 0.1% by weight, e.g. no more than 0.01% by weight compared to the weight of the particle.

The particle has a polypeptide shell, which typically surrounds the core. Where no water-immiscible liquid or other component is present in the core, the particle may, for example, have a hollow core, or the polypeptide shell may extend to the centre of the particle, i.e. the particle is formed only of the polypeptide shell. The nature of the polypeptide is not particularly limited and a wide range of different polypeptides can be used.

Typically, the polypeptide shell comprises a polypeptide having two or more cysteine residues. This enables the polypeptide to form disulphide cross-linking bonds in order to generate the polypeptide shell. Preferably, the polypeptide contains at least 4 cysteine residues, for example at least 10 cysteine residues. A greater number of cysteine residues, in particular cysteine residues which are accessible on the surface of the polypeptide, generates a greater degree of cross-linking in the polypeptide shell, which may in turn provide greater stability to the structure. Cysteine residues are naturally occurring in many proteins. However, cysteine residues may be introduced into the polypeptide chain, for example by genetic engineering techniques.

The polypeptide shell typically comprises a polypeptide having a molecular weight of at least 5kD, preferably at least 10kD. A wide range of polypeptides having varying molecular weights can be used to form the polypeptide shell. Typically, therefore, the molecular weight of the polypeptide is from 5 to 80kD, preferably from 10 to 50kD.

Typically, the polypeptide shell comprises a protein. In one embodiment, the polypeptide shell comprises a human blood protein (including fragments thereof). Examples of suitable proteins include albumin, insulin, globulin and haemoglobin. Of these, albumin, insulin and globulin are preferred. In one preferred aspect of this embodiment, the polypeptide shell comprises a serum albumin protein, preferably human serum albumin. Human serum albumin, and other blood proteins, are non-immunomodulatory, i.e. they do not give rise to an immune response on administration to a human. They are therefore beneficial where the polypeptide shell itself is not intended to generate an immunomodulatory response, e.g. where an adjuvant is present in the water-immiscible core and no further immunomodulatory response is desired.

In one embodiment, the polypeptide shell comprises one or more non-immunomodulatory polypeptides, typically one or more non-immunomodulatory proteins, i.e. one or more polypeptides or proteins which do not give rise to an immune response on administration to a human. In this embodiment, preferably the polypeptide shell contains no more than 5% by weight preferably no more than 1% by weight immunomodulatory protein, more preferably the polypeptide shell does not comprise an immunomodulatory protein. Preferably, in this embodiment, the polypeptide shell comprises one or more human blood protein, in particular one or more of albumin, insulin, globulin and haemoglobin, preferably human serum albumin. More preferably, in this embodiment, the polypeptide shell consists essentially of, in particular consists of, one or more human blood proteins, in particular one or more of albumin, insulin, globulin and haemoglobin. Preferably the polypeptide shell consists essentially of, in particular consists of human serum albumin.

In an alternative embodiment, the polypeptide shell comprises a polypeptide capable of generating an immune response, typically a pathogenic antigen protein and/or an adjuvant protein. In one embodiment, the polypeptide shell comprises at least one pathogenic antigen protein. The pathogenic antigen protein may be a full length protein or a fragment thereof. Examples of suitable antigen proteins include fragments of viral proteins or of parasitic proteins. The antigen may from a pathogen selected from Ca109 (flu), a coronavirus (e.g. Beta-coronaviridae or SARS-CoV-2), hepatitis B, *Bordatella pertussis* (whooping cough), *Streptococcus pneumoniae,* a meningococcus bacterium, human papilloma virus (HPV), or *Plasmodium* sporozoites (malaria parasite).

In one preferred aspect, the antigen protein is a viral spike protein, preferably a spike protein of the SARS-CoV-2 protein (e.g. SARS-CoV-2, S1 subunit protein (RBD)). In another aspect, the antigen is a circumsporozoite protein (CSP), a secreted surface protein of the sporozoite parasite. In a further aspect, the antigen is HepB surface antigen protein (HBsAg). In a further aspect, the antigen is associated with the influenza virus and is selected from haemagglutinin and/or an influenza neuraminidase. In another aspect, the antigen is filamentous haemagglutinin (pertussis). In another aspect, the antigen is pneumococcal surface protein A (PspA). In another aspect, the antigen is selected from Neisserial adhesin A (NadA), Neisserial Heparin Binding Antigen (NHBA) and/or factor H binding protein (fHbp). In another aspect, the antigen is an HPV-16 E6/E7 fusion protein. Fragments or genetically modified versions of any of these proteins may also be used.

The polypeptide shell of the particle may comprise two or more different antigen proteins. For example, two or more different antigen proteins of the same pathogen, or two or more antigens of different pathogens. Alternatively or additionally, a composition according to the invention may comprise two or more different particles, wherein the particles comprise different antigen proteins in their polypeptide shell. In this case, the composition may comprise particles having two or more different antigen proteins of the same pathogen, and/or two or more antigens of different pathogens.

In another embodiment, the polypeptide shell comprises an adjuvant polypeptide, such that the polypeptide shell may act as an adjuvant, increasing the immunomodulatory effect of the vaccine. Examples of suitable adjuvant polypeptides include non-human albumin proteins (e.g. bovine serum albumin, mouse serum albumin, ovalbumin), sFLT ligand, cytokines and chemokines such as interleukins (e.g. IL-1, IL-2, IL-4, IL-5, IL-6, IL-7, IL-12, etc), macrophage colony stimulating factor (M-CSF), tumour necrosis factor (TNF), and granulocyte and macrophage colony stimulating factor (GM-CSF). Ovalbumin is a preferred adjuvant protein. A combination of two or more such proteins may be used. Typically, where the polypeptide shell comprises an adjuvant polypeptide, the vaccine composition of the invention additionally comprises a separate vaccine, e.g. an RNA or DNA vaccine.

The polypeptide shell may comprise a single polypeptide, or a mixture of two or more different polypeptides. For instance, the polypeptide shell may comprise two or more different pathogenic antigen proteins. Alternatively, the polypeptide shell may comprise two or more different adjuvant polypeptides. Further, the polypeptide shell may comprise one or more pathogenic antigen protein and one or more adjuvant polypeptide. Preferably, the polypeptide shell comprises a single polypeptide (i.e. the polypeptide shell contains a single type of polypeptide, including fragments thereof, and does not contain a combination of two or more different polypeptides).

The polypeptide shell is optionally conjugated to one or more ligands. For example, the polypeptide shell may be conjugated to one or more ligands selected from a peptide, a protein, a sugar, a nanoparticle and a nucleic acid. Labelling moieties may also be conjugated to the polypeptide shell. Conjugation to proteins is commonly used for a variety of reasons. For example, the properties of the protein may be modified by conjugation to glycans (sugars) or to further proteins (e.g. antigens) having specific desired properties. In one embodiment, the polypeptide shell is conjugated to one or more adjuvants. Suitable adjuvants include an aluminum salt such as alum, aluminum hydroxide or aluminum phosphate, but may also be a salt of calcium (e.g. calcium phosphate hydroxide), iron or zinc, or may be an insoluble suspension of acylated tyrosine, or acylated sugars, or may be cationically or anionically derivatised saccharides, polyphosphazenes, biodegradable microspheres, monophosphoryl lipid A (MPL), lipopolysaccharides, lipid A derivatives (e.g. of reduced toxicity), 3-O-deacylated MPL [3D-MPL], detergents, e.g. quil A, Saponin, QS21, Freund's Complete Adjuvant, Freund's Incomplete Adjuvant (Difco Laboratories, Detroit, Mich.), Merck Adjuvant 65 (Merck and Company, Inc., Rahway, N.J.), AS-2 (Smith-Kline Beecham, Philadelphia, Pa.), CpG oligonucleotides, oils, e.g. squalene, mineral oils e.g. paraffin oil, food-based oils e.g. adjuvant 65 (derived from peanut oil), bacterial products, e.g. killed bacteria selected from *Bordatella pertussis, Mycobacterium bovis,* toxoids, bioadhesives and mucoadhesives, microparticles, liposomes, polyoxyethylene ether formulations, polyoxyethylene ester formulations, muramyl peptides or imidazoquinolone compounds (e.g. imiquamod and its homologues). Human immunomodulators including cytokines such as interleukins (e.g. IL-1, IL-2, IL-4, IL-5, IL-6, IL-7, IL-12, etc), macrophage colony stimulating factor (M-CSF), tumour necrosis factor (TNF), granulocyte and macrophage colony stimulating factor (GM-CSF) may also be used as adjuvants. Such adjuvants may be modified to include a suitable binding site to enable conjugation to the polypeptide shell.

A common technique for conjugation to proteins, which can be employed in the present invention, is to target lysine residues on the protein surface. Thus, ligands having a group reactive with amine (e.g. NHS) may be conjugated to lysine on the polypeptide shell. However, alternative conjugation chemistries may be employed.

The ligands may be conjugated to the polypeptide shell after particle formation. Alternatively, the ligand may be conjugated to the polypeptide before formation of the particle.

The shell thickness of the polypeptide particle is typically at least 10nm, preferably at least 20nm, for example about 30-40nm. For example, the polypeptide shell thickness may be from 10 to 100nm, preferably from 20 to 60nm. The thickness of the polypeptide nanoparticle shell can be determined by cryo-slicing and TEM imaging.

The particles of the invention are highly stable and can be stored as a suspension in aqueous solution or in solid form. For instance, the particles can be stored for at least a period of several months at 4°C.

### Synthesis

The polypeptide particles of the invention can be made by a two step process which involves (a) obtaining particles having a polypeptide shell and a water-immiscible core comprising one or more volatile components, optionally one or more non-volatile components and optionally one or more adjuvants, and (b) removing the volatile component from the core in order to create the desired indented particle having ultrasound-responsive properties.

The invention therefore provides a method of producing a polypeptide particle according to the invention, comprising:
- providing a water-immiscible phase comprising one or more volatile components, optionally one or more non-volatile components and optionally one or more adjuvants;
- mixing said water-immiscible phase with an aqueous solution of at least one polypeptide, wherein the polypeptide optionally comprises a pathogenic antigen protein and/or an adjuvant protein, thus providing a biphasic composition;
- cross-linking the polypeptide to generate a particle having a core comprising the water-immiscible phase and a shell comprising the at least one polypeptide; and
- creating one or more indentations in the particle by subjecting the particle to reduced pressure conditions, to remove volatile component from the core.

The particles are typically produced by first creating a biphasic composition comprising (1) a water-immiscible phase comprising one or more volatile components, optionally one or more non-volatile components and optionally one or more adjuvants; and (2) an aqueous phase comprising the polypeptide or mixture of polypeptides which is to be present in the polypeptide shell. The aqueous solution typically comprises at least about 0.05% w/v of polypeptide, for example at least about 0.5%, preferably at least about 1% w/v polypeptide. Typically the polypeptide concentration is no higher than about 25% w/v, e.g. no higher than about 15% w/v, preferably no higher than about 10 %w/v. Thus, suitable concentrations of polypeptide in the aqueous phase are from 0.5 to 15% w/v, preferably from 1 to 10% w/v, more preferably about 5% w/v.

The volumetric ratio of water-immiscible phase to aqueous phase in the biphasic mixture is typically about 1:5 to 3:1, for example from 1:4 to 1:1, e.g. about 1:3.

The water-immiscible phase comprises one or more volatile components. The nature of the volatile component(s) is not particularly limited, but is typically selected so that the or each volatile component is (a) water-immiscible, typically it is a volatile organic component, e.g. a volatile organic solvent, and (b) easily removed in the reduced pressure step. Typically, therefore, the or each volatile component has a vapour pressure at 25°C which is greater than that of water at the same temperature. Typically, the or each volatile component has a vapour pressure at 25°C of at least 3 kPa, preferably at least 3.5 kPa, for example at least 5 kPa, at least 10 kPa or at least 15 kPa. at 25°C. Suitable volatile components include hexane, chloroform, ethyl acetate, propyl acetate and toluene. Mixtures of two or more of these components can be used.

The volatile component may initially be used as a solvent for any adjuvant present, such that a solution of adjuvant in the volatile component(s) is used to produce the particles of the invention. Any pharmaceutically acceptable excipients which are used may also be dissolved in the volatile component(s).

The biphasic mixture may be held at elevated temperature for a period of time prior to the next step. For instance, the biphasic mixture may be incubated at around 37°C for up to 2 hours, for example for about 1 hour.

The biphasic mixture comprising the water-immiscible phase and aqueous phase is then subject to conditions which cause cross-linking of the polypeptides, typically by cross-linking cysteine residues in the polypeptide. The conditions used may also emulsify the mixture. Typically, for example, cross-linking is achieved by homogenising the bi-phasic mixture e.g. by applying high pressure and/or high shear stress conditions. Preferably, homogenisation is carried out by use of ultrasound. The high shear stress causes cross linking of the polypeptide, in particular by connecting cysteine groups within the polypeptide chains. It is understood that the homogenisation conditions may oxidise the sulfhydryl groups on a cysteine residue, or disrupt existing disulphide bonds, thereby allowing new disulphide bonds to form and generating the cross-linked shell structure. Water-immiscible phase remains physically trapped within the cross-linked polypeptide shell, providing the desired core-shell structure. The particles produced by the homogenisation step are generally substantially spherical in shape.

One example of a method for achieving homogenisation is to place an ultrasound horn at the interface of the aqueous and water-immiscible phases, and apply low-frequency ultrasound. Ultrasound may be applied at around 50% amplitude. For example, ultrasound may be applied for at least 1 minute, e.g. about 3 minutes. For example, particle formation has been achieved using a QSonica Q125 ultrasound probe (frequency: 20kHz; power: 125 watts) at 50% amplitude for 3 minutes.

An alternative method for achieving homogenisation is to use hydrodynamic cavitation, which uses high pressure to pump a liquid through a narrow orifice. The increase in flow velocity as the liquid passes through the orifice, and subsequent decrease in velocity once past the orifice, causes cavitation nuclei to grow and then collapse. Applying hydrodynamic cavitation to the biphasic mixture may cause cross-linking of cysteine residues and/or emulsification, leading to formation of particles. Hydrodynamic shearing is a further alternative method which may produce the same reactive oxygen species that reduce / oxidise cysteine residues that lead to particle formation. Other methods of sonication may also be used.

Spherical particles having a water-immiscible core and a polypeptide shell, which are produced by such homogenisation methods, are known in the art, and methods for their synthesis have been previously described (see Suslick K. S. and M. W. Grimstaff, Journal of the Americal Chemical Society, 1990, 112(21), p7807-7809, the contents of which are incorporated herein by reference). The particles of the invention may be made by such techniques or other methods of sonication, such as those described in US5439686, the contents of which are incorporated herein by reference.

Following the creation of the polypeptide shell, the particles of the invention undergo a step in which the volatile component(s) are removed, causing deformation of the particle and thus the desired indented structure. Removal of the volatile component(s) can be done under reduced pressure conditions. For instance this step can be carried out using a vacuum protein concentrator such as a Speed Vac^{™} (Thermo Scientific), but other instrumentation such as a rotary evaporator can be used. Reduced pressure conditions are typically applied for at least 30 minutes, preferably at least an hour. For instance, reduced pressure may be applied for from 2 to 16 hours.

The nature and amount of volatile component which is present in the water-immiscible phase affects the particle size and may be used as a factor in controlling the size of particle produced. The amount of volatile component also affects the deformation of the particle and thus the size of any indentation. In general, the greater the amount of volatile component, the greater the size of the indentation which is expected to be produced. Variation in the size of the indentation may also be relevant to the cavitation effects generated by the indented particle, due to the change in the potential for gas bubbles to be trapped within the cavity of the indentation.

Where both volatile and non-volatile components are used in the water-immiscible phase, the amount of volatile component can be varied by changing the relative amount of non-volatile and volatile component in the water-immiscible phase. Typically, the volume ratio of (a) volatile component optionally containing dissolved adjuvant, to (b) non-volatile component is from about 1:20 to 20:1, preferably from about 1:4 to 15:1, e.g. about 1:3 to 3:1, more preferably about 0.5:1 to 2:1 (1:2 to 2:1), most preferably about 1:1. A minimum volume ratio of at least 1:20 of volatile to non-volatile component (about 5% non-volatile component) is typically needed to achieve indentation in the particle on removal of the volatile component form the core. A volatile content of at least 40% by volume, preferably from 45 to 55% by volume in the water-immiscible phase, preferably around 50% by volume (or 1:1), has been found to provide improved cavitation properties.

Alternatively, the water-immiscible phase may comprise substantially no non-volatile component, or non-volatile component may be absent. Thus, there may be no more than 5% by volume, for example no more than 2%, 1% or 0.5% by volume non-volatile component, for example no more 0.1% or 0.01% by volume non-volatile component.

The amount of adjuvant which is present in the water-immiscible phase, where used, may vary and depends on the nature of the agent. For instance, the adjuvant may be present in an amount of from 0.1ug to 1mg/ml, typically from 0.5 to 100ug/ml, e.g. from 1 to 10ug/ml in the volatile component. The amount of adjuvant which is present in the core of the particle once produced can be controlled by varying the concentration of the agent in the volatile component.

The method of the invention may further comprise the steps of:
- drying the particle; and
- optionally re-suspending the particle in a liquid medium.

The drying step may be carried out by evaporation, e.g. under reduced pressure and/or with mild heating. For instance this step can be carried out using a rotary evaporator set to a temperature of up to about 40C. Typically, the drying step is combined with the step of evaporation of volatile component, but continuing the reduced pressure (and optionally mild heat) conditions for a longer period of time and by use of mild heating, in order to evaporate water to provide a solid product. Thus, for instance, removal of volatile component and drying may be carried out by applying reduced pressure conditions and heating at up to 40C until a solid is produced.

The particles are then preferably re-suspended in a liquid medium, typically water or an aqueous solution such as a buffer. Re-suspension may provide improved long term stability to the particles and they are therefore preferably re-suspended prior to storage. However, particles may alternatively be stored in solid form and re-suspended prior to use. Drying and re-suspension may assist in trapping gas bubbles in any indentations on the particle, which provides improved inertial cavitation response.

The method may also comprise:
- lyophilising the particle; and
- optionally re-suspending the particle in a liquid medium.

Typically, the particles are re-suspended in a liquid medium, typically water or an aqueous solution such as a buffer. Re-suspension may provide improved long term stability to the particles and they are therefore preferably re-suspended prior to storage. However, particles may alternatively be stored in solid (lyophilised) form and re-suspended prior to use. Lyophilisation and re-suspension may further assist in trapping gas bubbles in any indentations on the particle, which provides improved inertial cavitation response. A particularly improved cavitation response has been demonstrated with particles which have been both dried and re-suspended, and subsequently lyophilised and re-suspended.

The additional steps of drying and lyophilisation may be carried out with any of the particles described herein. However, in a preferred embodiment, where the particle is produced by use of only volatile component(s) in the core (i.e. the final particle after evaporation of volatile component does not comprise a water-immiscible liquid in the core), or where the particle is produced using a low proportion, typically less than 10% by volume, preferably less than 5% by volume, e.g. less than 1% or less than 0.1% by volume, of non-volatile component in the water-immiscible phase (such that the final particle has a core comprising a small amount of non-volatile component, e.g. less than 10 wt%, preferably less than 5 wt% compared to the total particle), it is preferred that the particles are dried and/or lyophilised. Most preferably, these particles are dried, re-suspended and then lyophilised. Yet more preferably, these particles, are dried, re-suspended, lyophilised and again re-suspended.

The particles can be purified by suitable means and/or subject to size filtration. For example, particles can be separated from residual oil, solvent and protein by any suitable means, for example, by dialysis e.g. through a dialysis filter having a molecular weight cut off of appropriate size (e.g. 1M Da). Size filtration and/or centrifugation may also be used to limit the maximum size of the particles. Purification may be carried out following evaporation of the volatile component, following drying and re-suspension, or following lyophilisation and resuspension.

The particles may be suspended in a liquid medium for storage or use, e.g. water, or an aqueous solution, optionally a buffer solution.

### Compositions

The present invention provides a composition, typically a pharmaceutical composition, comprising one or more polypeptide particles according to the invention. Compositions which contain a vaccine are referred to herein as vaccine compositions (in particular transdermal vaccine compositions). Compositions which contain an adjuvant but do not contain a vaccine are referred to herein as adjuvant compositions. The present invention provides both transdermal vaccine compositions and adjuvant compositions for transdermal administration. The composition may comprise a plurality of the polypeptide particles alone, or the particles may be provided together with one or more pharmaceutically acceptable carriers or diluents. Typically, compositions provided together with a carrier or diluent contain up to 85 wt% of a particle of the invention. More typically, such a composition contains up to 50 wt% of a particle of the invention. Preferred pharmaceutical compositions are sterile and pyrogen free.

The compositions of the invention may comprise one or more different types of particle according to the invention. For instance, the composition may comprise first particles having a first type of polypeptide shell and second particles having a second (different) type of polypeptide shell. Thus, for instance, the compositions may comprise particles comprising two or more particles comprising two or more different antigen proteins relating to the same pathogen. Alternatively or additionally, the composition may comprise particles comprising pathogenic antigen proteins and separate particles comprising adjuvant. Alternatively or additionally, the composition may comprise particles comprising antigen proteins relating to different antigens, such that the composition may be used to vaccinate subjects against two or more different pathogens. Typically, the compositions of the invention comprise a single type of particle.

The compositions may comprise particles which themselves include an adjuvant. Alternatively or additionally, further adjuvant(s) may be included in the composition. Suitable further adjuvants include an aluminum salt such as alum, aluminum hydroxide or aluminum phosphate, but may also be a salt of calcium (e.g. calcium phosphate hydroxide), iron or zinc, or may be an insoluble suspension of acylated tyrosine, or acylated sugars, or may be cationically or anionically derivatised saccharides, polyphosphazenes, biodegradable microspheres, monophosphoryl lipid A (MPL), lipopolysaccharides, lipid A derivatives (e.g. of reduced toxicity), 3-O-deacylated MPL [3D-MPL], detergents, e.g. quil A, Saponin, QS21, Freund's Complete Adjuvant, Freund's Incomplete Adjuvant (Difco Laboratories, Detroit, Mich.), Merck Adjuvant 65 (Merck and Company, Inc., Rahway, N.J.), AS-2 (Smith-Kline Beecham, Philadelphia, Pa.), CpG oligonucleotides, oils, e.g. squalene, mineral oils e.g. paraffin oil, food-based oils e.g. adjuvant 65 (derived from peanut oil), bacterial products, e.g. killed bacteria selected from *Bordatella pertussis, Mycobacterium bovis,* toxoids, bioadhesives and mucoadhesives, microparticles, liposomes, polyoxyethylene ether formulations, polyoxyethylene ester formulations, muramyl peptides or imidazoquinolone compounds (e.g. imiquamod and its homologues). Human immunomodulators suitable for use as adjuvants include cytokines such as interleukins (e.g. IL-1, IL-2, IL-4, IL-5, IL-6, IL-7, IL-12, etc), macrophage colony stimulating factor (M-CSF), tumour necrosis factor (TNF), granulocyte and macrophage colony stimulating factor (GM-CSF).

The transdermal vaccine compositions may comprise particles which themselves include a pathogenic antigen protein (e.g. in the polypeptide shell or as a ligand attached to the polypeptide shell). Alternatively, or additionally, a separate vaccine may be included in the composition. The separate vaccine may be a pathogenic antigen protein or a DNA or RNA vaccine. Preferably, the transdermal vaccine composition comprises particles comprising a pathogenic antigen protein in the polypeptide shell and/or the composition further comprises a DNA or RNA vaccine.

The particles in the composition may be substantially monodisperse. For instance, they may have a polydispersity index of 0.20 or less, e.g. 0.19 or less, 0.18 or less, 0.17 or less, 0.16 or less, or 0.15 or less. Greater monodispersity of the particles may provide a greater control over cavitation and thus improved acoustic tuning. Polydispersity may be determined by DLS.

In one embodiment, a composition provided together with a carrier or diluent is a liquid dispersion. Typically, water (preferably sterile water) is used as diluent for a liquid dispersion.

The particles of the invention may be administered transdermally, for example as a gel. A gel for transdermal administration may comprise a diluent selected from an alcohol such as ethanol or propanol, glycerine, propylene glycol or polyethylene glycol. The gel may also comprise preservatives and may comprise water or a buffer solution. Permeation enhancers such as fatty acids, fatty alcohols, urea, sulphoxides, azone, pyrrolidones, essential oils, terpenes and terpenoids may be incorporated. Alternatively the gel for transdermal delivery may be formulated as a proniosome or microemulsion gel.

### Administration

The particles or compositions of the invention are typically applied to an area of skin, and ultrasound provided to the area in order to generate the inertial cavitation response of the particles.

Ultrasound is typically delivered at a frequency of from 100 to 1000kHz, preferably from 100 to 500kHz, for example from 200 to 300kHz and optionally a duty cycle (DC) of from 1% to 20%, preferably from 5% to 20%, e.g. from 5% to 15%. Typically, the ultrasound delivered has a pulse repetition frequency (PRF) of from 0.01 to 100 Hz, preferably from 0.1 to 100 Hz, e.g. from 1 to 50Hz, preferably from 5 to 20Hz. Typically, the ultrasound is provided at a peak negative pressure (PNP) of 0.5 MPa to 3.0 MPa, preferably a PNP of from 0.5 MPa to 2.0 MPa, e.g. from 1.0 to 2.0 MPa .

Particularly preferred ultrasound conditions are from 1.0 to 2.0MPa and 100 to 500kHz, e.g. 1.8 MPa and 265kHz.

Ultrasound may be applied for a duration of from 10 seconds to 15 minutes, typically from 30 seconds to 10 minutes, preferably from 1 to 5 minutes.

Application of ultrasound to a formulation of particles applied to the skin may cause inertial cavitation within the formulation, assisting in the transdermal delivery of the particles, and any co-administered agent such as a co-administered vaccine. Ultrasound has previously been used to assist transdermal delivery of drugs. However, previous work has focussed on the use of ultrasound to permeabilise the skin and particles for delivery have been administered with additional means, for example via ballistic delivery. In contrast, the present invention enables particles to travel through the skin solely by applying the particles in a suitable formulation, such as a gel, and providing ultrasound at the site. The combination of the small size of the particles, and the microstreaming effect caused by inertial cavitation, pushes the particles, together with any co-delivered agent, through the relatively impermeable stratum corneum, thus providing effective transdermal delivery.

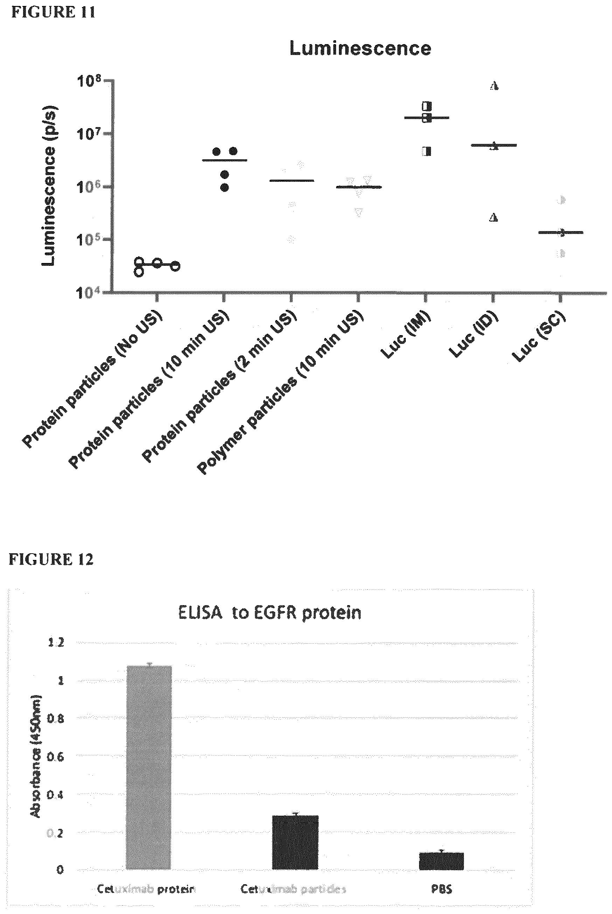

The examples provided herein demonstrate the successful administration of the particles of the invention via transdermal delivery. Particles were administered together with DNA encoding luciferase and post-administration luminescence measurements provided an indication of the degree of successful transdermal administration (see Figure 11). Luminescence was comparable to that achieved by direct injection of DNA.

### Use of Particles

The particles and compositions of the invention may be used in a method of vaccination. The ability of the particles of the invention to elicit antibodies to the polypeptide incorporated into the polypeptide shell provides a particular benefit for use in a method of vaccination. The particles may alternatively, or additionally, be provided with a separate vaccine, e.g. an RNA or DNA vaccine.

Accordingly, the present invention provides a particle or composition as described herein for use in a method of vaccination. Also provided is a method of vaccination which comprises administering an effective amount of a particle or composition as described herein to a subject. Also provided is the use of a particle or composition as described herein in the manufacture of a medicament for use in a method of vaccination.

Particles which themselves generate an immunologic effect as a vaccine, may be provided together with an adjuvant and/or the particles themselves may additionally comprise an adjuvant. In one aspect, the particles comprise an adjuvant in the water-immiscible core of the particle. However, given the increased immunologic effect of the particles, an advantage of the use of the particles in a method of vaccination is that a sufficient immunologic effect may be achieved without administration of an adjuvant.

In alternative embodiments of the invention, the particles are administered together with (or the transdermal vaccine composition of the invention may comprise) a vaccine, e.g. a (ribo)nucleic acid vaccine. In some embodiments, the nucleic acid vaccine is an RNA or DNA vaccine. In this embodiment, the particles of the invention typically comprise an adjuvant.

Where an adjuvant is used in the method of vaccination, the adjuvant may be provided separately from the particles of the invention (i.e. in a separate composition), it may be provided in the same composition as the particles (but not as part of the particles themselves) or an adjuvant may be incorporated into the particles themselves. Two or more of these approaches may be combined.

Where an adjuvant is present in the particle itself, it may be comprised within the water-immiscible core, and/or an adjuvant polypeptide may be comprised within the polypeptide shell and/or an adjuvant may be provided as a ligand which is conjugated to the polypeptide shell of the particle of the invention.

In a particular aspect of the invention, the polypeptide particle or composition of the invention comprises an adjuvant (which may be present in the water-immiscible core, in the polypeptide shell, and/or as a ligand which is conjugated to the polypeptide shell) and the particle is administered together with, or the composition comprises, a vaccine, for example a (ribo)nucleic acid vaccine, in particular n RNA or DNA vaccine. Preferably, the polypeptide particle or composition of the invention has a polypeptide shell which comprises an adjuvant polypeptide as described herein, and the particle or composition is administered together with a vaccine, for example a (ribo)nucleic acid vaccine, in particular an RNA or DNA vaccine.

The present invention therefore also provides a composition or kit comprising particles of the invention, together with a vaccine, e.g. a (ribo)nucleic acid vaccine, typically an RNA or DNA vaccine. The particles typically comprise an adjuvant, preferably the particles have a polypeptide shell which comprises an adjuvant polypeptide.

### Dosages

The dose of particles of the invention may be determined according to various parameters, especially according to the substance used; the age, weight and condition of the individual to be treated; and the required regimen. The amount of particle in each dose may, for example, be selected as an amount which induces an immune response. A physician will be able to determine the required dosage for any particular individual. The dose may be provided as a single dose or may be provided as multiple doses, for example taken at regular intervals, for example 2, 3 or 4 doses. Typically polypeptide-based treatments are administered in the range of 1 pg to 1 mg, more typically 1 pg to 10 µg for particle mediated delivery and 1 µg to 1 mg, more typically 1-100 µg, more typically 5-50 µg for other routes. Generally, it is expected that each dose will comprise 0.01-3 mg of polypeptide. An optimal amount for a particular treatment can be ascertained by studies involving observation of immune responses in subjects.

### Examples

### Particle Synthesis

Ultrasound-responsive particles were prepared according to protocol A as follows:
3ml of a 50 mg/ml solution of protein is overlaid with 1ml of a water immiscible solution containing an oil, and optionally a volatile component and in some cases an additional agent, to produce a biphasic solution. The biphasic mixture is sealed in a glass vial and placed in an incubator at 37C for 1 hour.

An ultrasound horn is then placed at the interface of the two layers and low frequency ultrasound at 50% amplitude is applied for 3 minutes at 125 watts using QSonica Q125 at 50% amplitude. This produced an emulsion of particles comprising a core containing the water immiscible mixture and a protein shell.

The volatile solvent component of the particle core is then removed under reduced pressure using a SpeedVac^{™} protein concentrator to provide a solution containing the particles.

The particles are then separated from residual oil, solvent and protein by dialysis in a 1M Da molecular weight filter for 24 to 48 hours.

Particles were also produced according to a modified protocol B. Protocol B is identical to protocol A with the exception that the protein was at a lower concentration of 0.5mg protein in 666ul of water which was then overlaid with 333ul of the oil/volatile solvent layer.

Particles were also produced according to a modified protocol C:
3ml of a 50 mg/ml solution of protein is overlaid with 1ml of a water immiscible solution to produce a biphasic solution. The biphasic mixture is sealed in a glass vial and placed in an incubator at 37C for 1 hour.

An ultrasound horn is then placed at the interface of the two layers and low frequency ultrasound at 50% amplitude is applied for 3 minutes at 125 watts using QSonica Q125 at 50% amplitude. This produced an emulsion of particles comprising a core containing the water immiscible mixture and a protein shell.

The volatile solvent component of the particle core is then removed under reduced pressure by rotary evaporation at 40C for 48 hours to provide solid particles.

Particles are re-suspended in water. Some particles are then freeze-dried for 48 hours, followed by re-suspension in water once again.

The following particles were produced:

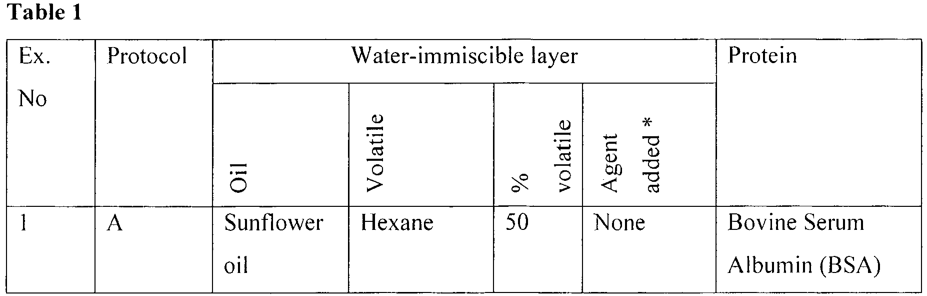

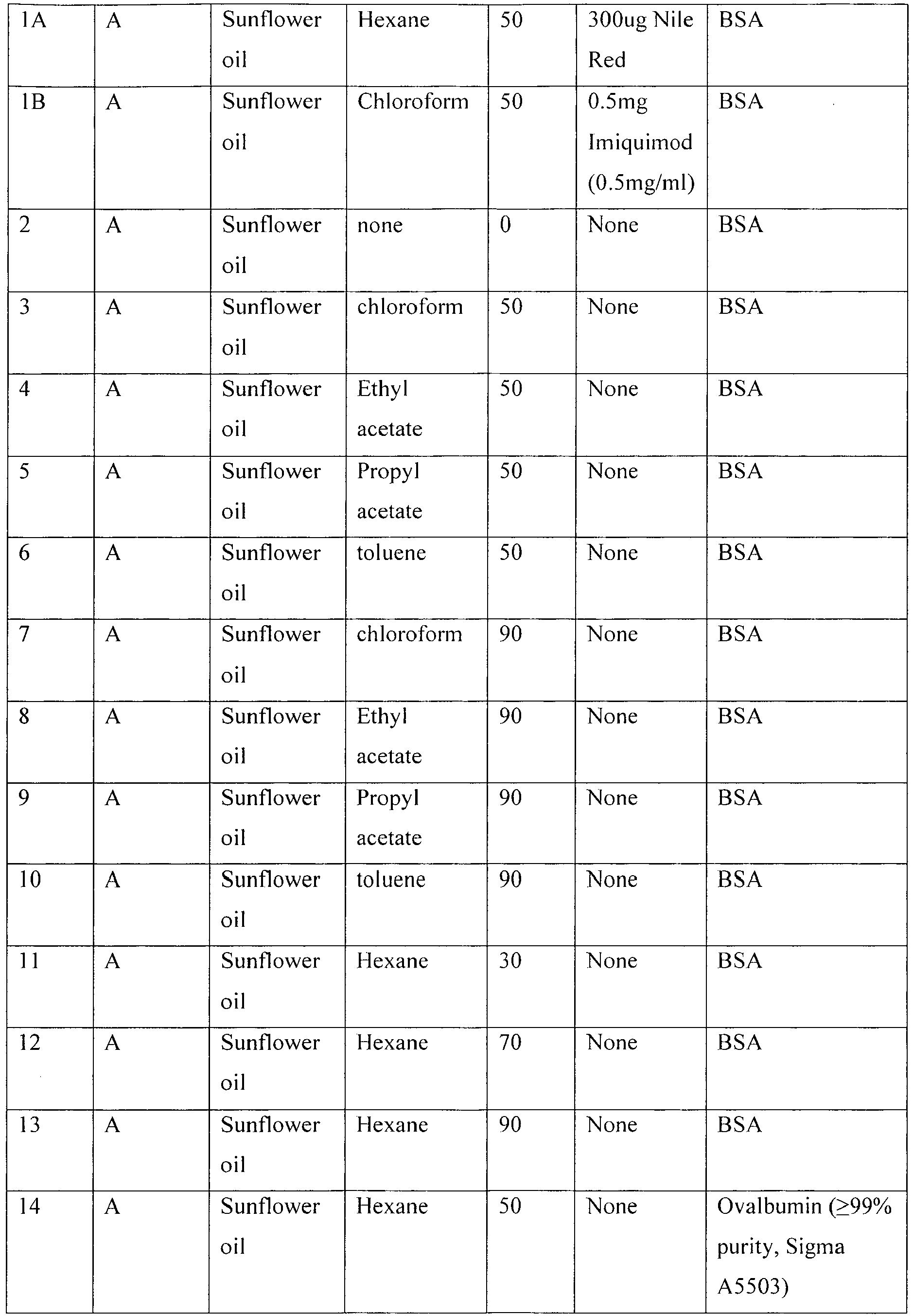

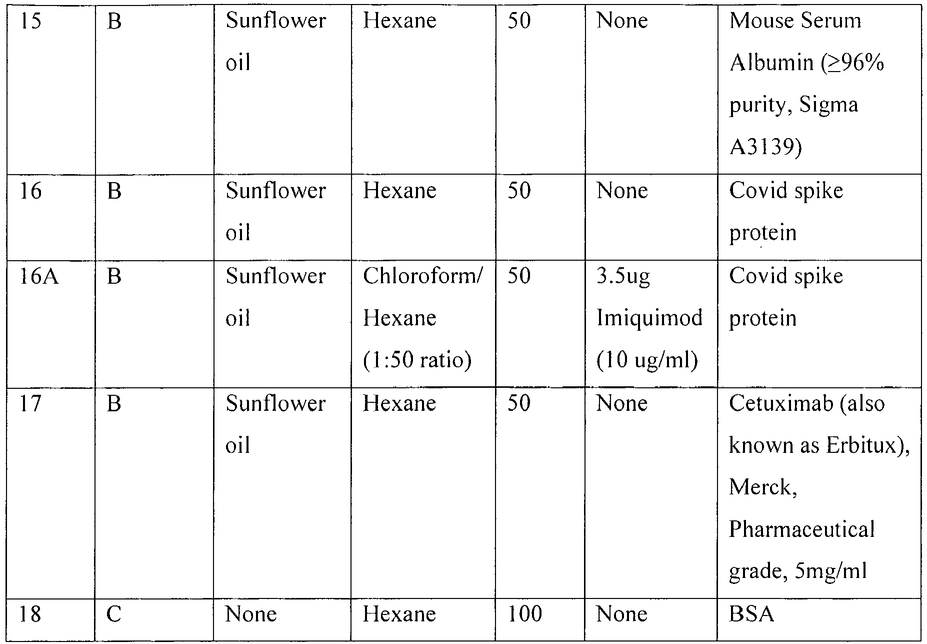

Bovine serum albumin (BSA) used was ≥99% purity, Sigma A7638.
Covid spike protein used was SARS-CoV-2, SI, RBD, Cambridge Bioscience, 230-30162-1000

*Amount agent added or concentration is the amount/concentration in the water-immiscible core as a whole.

### Particle Analysis and Chemical Modification of Particle Surface

Particles were analysed by dynamic light scattering (DLS), scanning electron microscopy (SEM) and via spinning disc confocal microscopy following conjugation of the particle to fluorophores.

### SEM analysis

### Instrumentation:

▪ Scanning electron microscopy (SEM) analysis was performed using a Zeiss Sigma 300 Field Emission Gun Scanning Electron Microscope (FEG-SEM).

### Method:

▪ Typically a 1 in 1000 dilution of a stock solution was made. This is fixed with 1% glutaraldehyde for 2h, and 10ul added to a black carbon tape and coated with gold before imaging

### Spinning disc confocal microscopy

### Instrumentation:

▪ An Andor Dragonfly spinning disc confocal microscope was used to generate high resolution Z stacks that were then compiled into 3D reconstructions of protein particles

### Method:

▪ Particles were reacted with CY-5^{®} N-hydroxysuccinimide (NHS) or AlexaFluor^{™} 488 N-hydroxysuccinimide (NHS) for 1h. Dialysis for 16h using a 10kDa molecular weight cassette was used to remove residual unconjugated dye prior to imaging.

### Dynamic Light Scattering

### Instrumentation:

▪ Size distributions were measured using DLS (ZetaSizerNano, Malvern) with a red laser. Each sample was tested with three measurements of approximately 10 runs.

### Method:

▪ 10ul of a particle sample prepared as set out above was diluted to 1 ml with Millipore water and analysed in a standard 1ml cuvette.

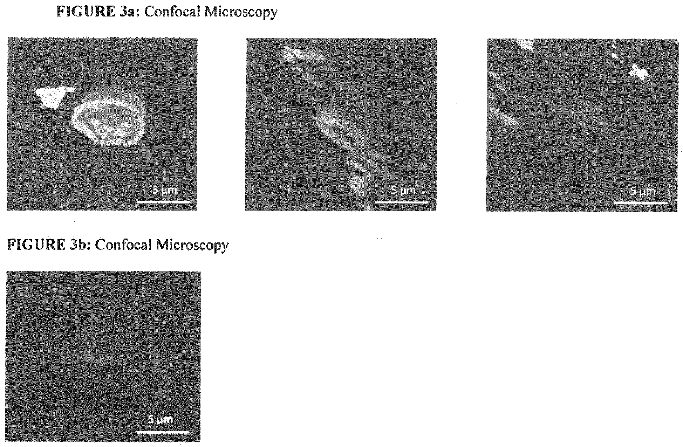

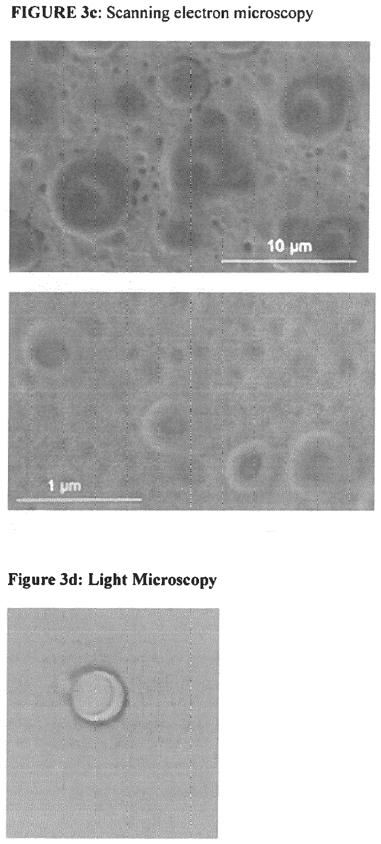

Figures 3a to 3b and 3c show the particles of Example 1 above via 3D confocal microscopy and SEM respectively. Ligands successfully conjugated to particles via NHS-lysine conjugation, and provided visibility of particles via confocal microscopy as shown in Figure 3a.

The particles can be seen to have an indented structure, consistent with the ability of the particle to trap a gas bubble in the indentation. The particles have either one or two major indentations.

This experiment also confirms that upon forming particles, the protein retains the ability to undergo standard protein conjugation reactions, in this case reaction with chemistry targeting lysine residues. The particles became fluorescent following conjugation of two different lysine reacting dyes.

Figure 3d shows a particle according to Example 18 (following freeze-drying and resuspension in water) viewed using light microscopy.

### Addition of Compound to Oil Core

Example 1A demonstrates that compounds dissolved in the oil layer during synthesis are incorporated into the oil core of the particles.

Protocol A was followed to produce the particles as set out in Example 1 above, but 300ug of Nile Red oil soluble fluorophore was incorporated into the oil layer prior to application of ultrasound to form the particles. Any residual fluorophore was removed from the particles by dialysis.

Fluorescent confocal microscopy was used to image the particles. This confirmed the presence of Nile Red inside the core of the particles.

Example 1B describes the production of particles containing the adjuvant imiquimod. imiquimod was included in the chloroform volatile component at a concentration of 1 mg/ml and mixed with the sunflower oil prior to particle production. Protein articles were produced according to the standard protocol A.

Purified protein particles were digested with beta mercaptoethanol and proteinase K for 1 hour at 60C to break up the external protein shell and release the inner core. The core content was then analysed by UPLC to confirm the presence of imiquimod. UPLC analysis was carried out substantially as described in the method by Balireddi et al (Hournal of Chrom Science, Volume 57, Issue 3, March 2019, p249-257).

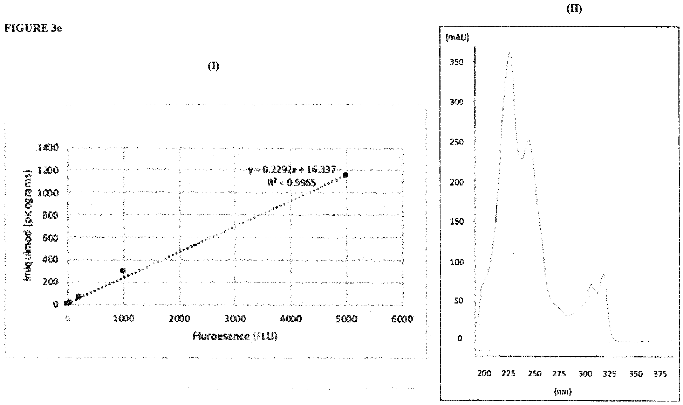

UPLC analysis confirmed the presence of imiquimod as shown in Figure 3e. Fig 3e(I) shows the imiquimod standard curve and the amounts of imiquimod detected in the particle core. Amounts in excess of 1 ug/ml were detected. Fig 3e(II) shows the UV absorbance profile, which corresponds to the characteristic absorbance profile of imiquimod.

### Effect of Volatile Component on Particle Size

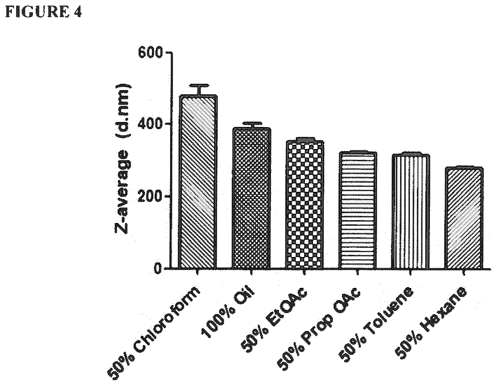

The nature of the volatile component was varied in Examples 3 to 6, in order to determine the effect on the particle size. Results are depicted in Figure 4, showing that variation of the volatile component can be used to vary the particle size. Similarly, the amount of the volatile component was varied in Examples 7 to 10, and the change in particle size studied. The results are depicted in Figure 5. Increase in the amount of volatile component generally leads to a reduction in particle size.

### Variation of Polypeptide, optionally with adjuvant in oil core

Variation of the protein to incorporate a variety of different proteins into the particle was achieved as set out in Examples 14 to 17. In particular, Example 14 demonstrates the production of a particle having an adjuvant protein, ovalbumin, in the polypeptide shell, whilst Example 16 demonstrates the manufacture of particles having a pathogenic antigen protein, covid spike protein, in the polypeptide shell.

Example 16A demonstrates the production of particles having covid spike protein as the polypeptide shell, and also having adjuvant in the water-immiscible core. In this example, protocol B was followed, and modified as follows:
Imiquimod was dissolved in chloroform at 0.5 mg/ml. 7ul (1 in 50 dilution) was then added to 326ul of the hexane/oil layer to provide a total volume of 333ul of oil layer. This oil layer was then overlaid on a solution of Covid spike protein to provide the biphasic mixture. Particles were produced in accordance with protocol B. The maximum content of imiquimod was therefore 1ug/100u1 in the resulting particle suspension.

### In Vitro Cavitation of Particles

All measurements of inertial cavitation were carried out at a frequency of 0.5 MHz, 1000 cycle pulse length, and 100 ms pulse repetition period, with varying driving pressures as set out below.

First, to characterise the cavitation ability of particles across a range of sizes, particles produced according to Example 1 above were filtered through 200nm and 450nm filters and then assessed for inertial cavitation. The passive cavitation detection (PCD) level was assessed at driving pressures of 0.5MPa, 1.0MPa, 1.5MPa and 2.0MPa. Time averaged PCD levels over a 30 second exposure are provided in Figure 6 for particles without size filtration (all sizes), and particles filtered through 200nm and 450nm filters. Cavitation was detected in all particles.

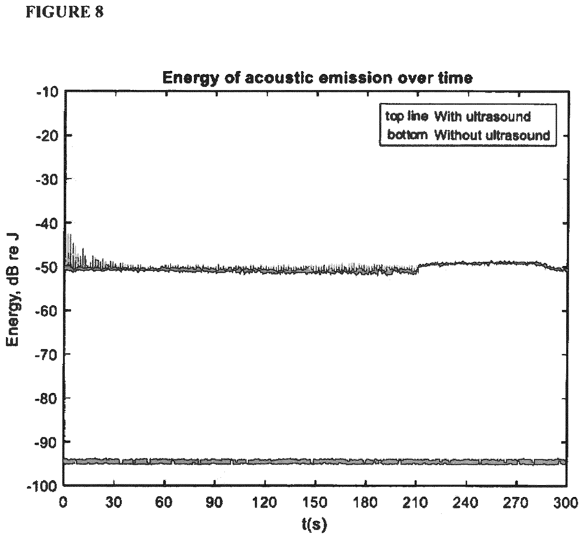

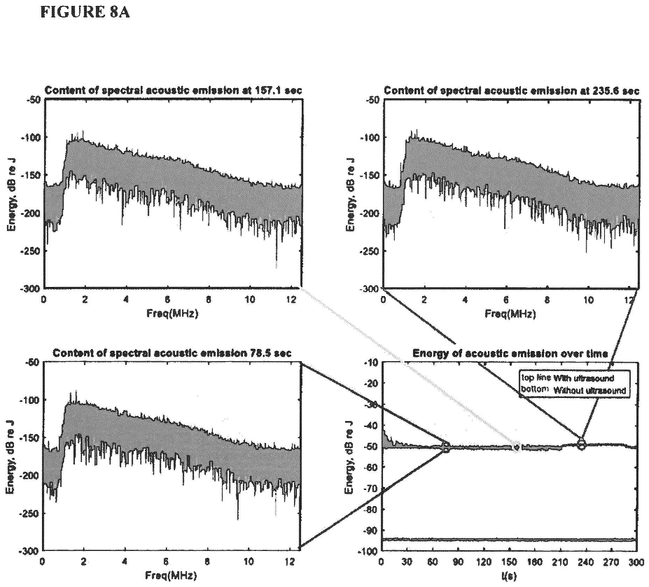

Inertial cavitation was also demonstrated, using particles produced according to Example 18 above. Particles were exposed to 181mVpp (equivalent to 1.2 MPa peak pressure) for 5 minutes. Figure 8 shows a representative energy trace demonstrating persistent inertial cavitation.

Inertial cavitation was also demonstrated using particles produced according to Example 18 above. Particles were exposed to 1.4M Pa, 1.7 MPa and 2.1MPa for at least 300 seconds. Particles were studied both before and after freeze-drying. Water was used as a control. Cavitation was observed under all conditions for all particles. At 1.4 MPa, cavitation was observed for less than 20s for dried but non-freeze-dried particles, but for over 150s for freeze-dried particles. At 1.7 MPa, cavitation was observed for around 100s for dried but non-freeze-dried particles, but for over 150s for freeze-dried particles. At 2.1 MPa, cavitation was observed for around 200s for dried but non-freeze-dried particles, but for over 300s for freeze-dried particles.

### Effect of Percentage Volatile Component in Core on Cavitation Ability

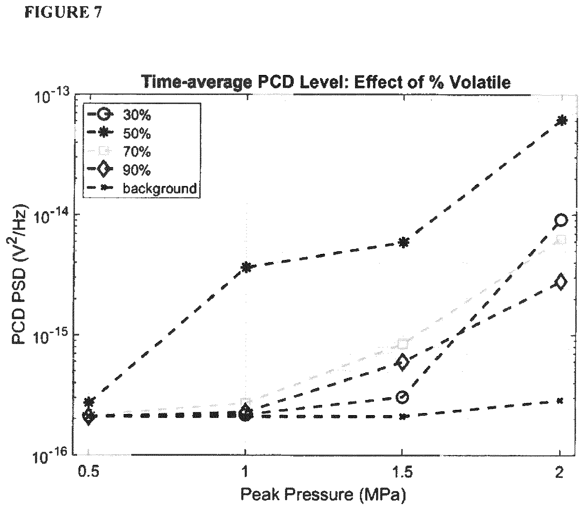

Examples 11 to 13 above describe the production of particles in an identical manner to Example 1 but using differing amounts of the volatile component, hexane. Experiments were conducted to establish if varying the ratio of volatile solvent (and as such the potential depth of the indentation in the particle) can affect the cavitation ability of the particle. Cavitation was studied in the same manner as set out above and the results are set out in Figure 7. Varying the volatile component (and thus the depth of the indentation) had a significant effect on the cavitation potential of the particles. 50% by volume volatile was optimum for cavitation of the resulting particle, although cavitation was detected at all volatile content levels.

### Ability of Particles to retain functionality of protein from which they are derived

The aim of this study was to assess the ability of particles to retain functional binding to a receptor following particle formation.

Particles of Example 17 containing cetuximab in the polypeptide shell (prepared according to protocol A as set out above) were dialysed for 48h using a 1M Da molecular weight filter to remove any free (non-particle bound) Cetuximab. An ELISA specific to the protein EGFR (a validated binding target for Cetuximab) was undertaken to compare the binding efficiency of equal amounts (mg/mg) protein of native Cetuximab to Cetuximab particles.

The results are depicted in Figure 12. Figure 12 demonstrates that particles composed of Cetuximab retain an ability to bind to EGFR. This supports the understanding that antigen proteins or adjuvant proteins forming part of the polypeptide shell are able to retain their therapeutic efficacy after particle formation. The reduced level of binding compared to native Cetuximab in this experiment is understood to relate to steric hindrance of the particles, and a lack of optimisation.

### In Vivo Cavitation Ultrasound Methodology

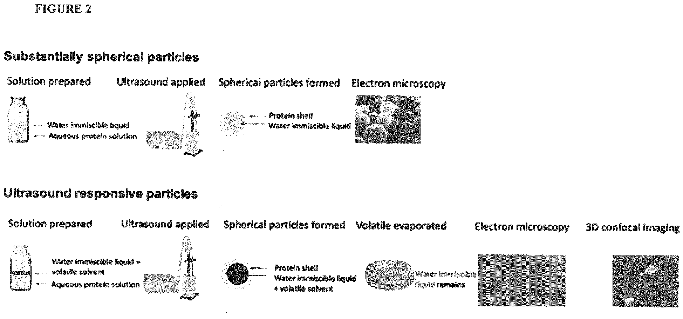

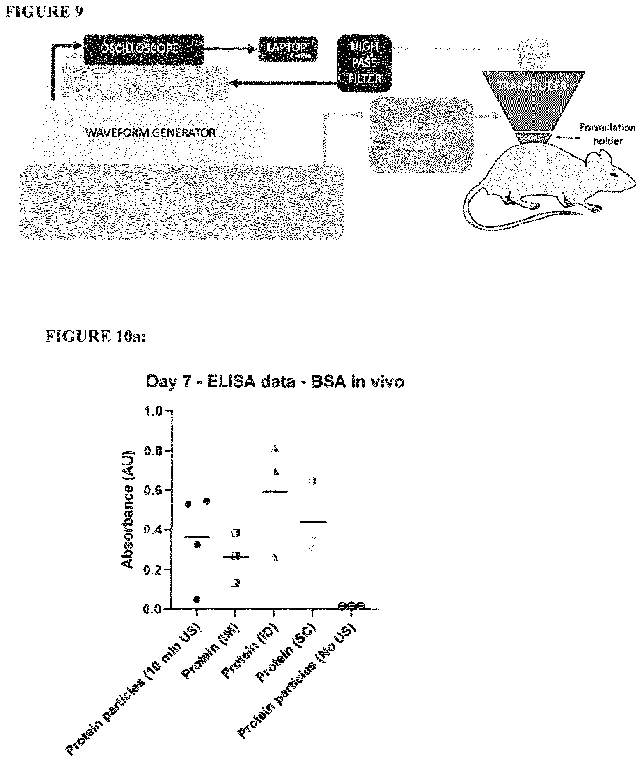

In vivo transdermal particle cavitation in the experiments described below was carried out by using a source transducer (117D-01, Sonic Concepts, USA) to initiate cavitation. The transducer was fitted with a Perspex coupling cone (used to efficiently transmit ultrasound from the source to the therapy target), filled with degassed water and covered with an acoustically transparent Mylar plastic. The coupling cone shape was designed to align with the outer envelope of the focused ultrasound beam. A formulation holder, containing particle solution, was placed on the murine skin and the source transducer was placed on top of this, with the Mylar plastic acoustic window positioned to prevent air entering the acoustic path. A single element spherically focused ultrasound transducer acting as passive acoustic detector (PCD) was coaxially located inside the source transducer, to allow the cavitation signal to be recorded. The PCD signal was passed through a 2.0 MHz high pass filter and amplified with a gain of 25 by a pre-amplifier (SR445A, Stanford Research Systems, USA). The signal was then digitised and monitored in real time by an 12-bit digital oscilloscope (Handyscope HS3 100 MHz, TiePie Engineering, The Netherlands) and the data was saved on an external hard drive (Portable SSD T5, Samsung, Korea) (Figure 2). This specific cavitation detection setup has been documented in Gray et al, IEEE Trans. UFFC 65(2) 2018, pp 258-267. The set-up is schematically depicted in Figure 9.

### Transdermal Particle Delivery and In Vivo Cavitation

Particles according to the invention were applied to mice to study the transdermal delivery of the particles. Immune response to the protein delivered was used to assess successful delivery of particle via transdermal administration followed by ultrasound.

8 mice were used in the experiment, and each mouse received two separate particle preparations, one applied to each flank. Thus, 16 experiments were carried out in total as follows:
Group 1: 4 x Protein particles + US
Group 2: 3 x Protein particles without US
Group 3: 3 x protein subcutaneous injection
Group 4: 3 x protein Intradermal injection
Group 5: 3 x protein Intramuscular injection

Protein particles used were particles produced in accordance with Example 1 above. The particles were administered to each mouse either by direct application to the mouse flank (with or without application of ultrasound), or by injection as indicated above. The total protein content of each component administered was 9mg. It is important to note that for injection, the full 9mg is delivered to the mouse, whereas for transdermal administration, only a fraction of the 9mg protein is delivered. Where ultrasound was used, this was carried out as discussed in *"In Vivo Cavitation Ultrasound Methodology"* above. Exposure time was 10 minutes.

Following administration of the particles in the designated manner for each group, immune response to BSA protein was determined by carrying out ELISA analysis to study immune response to BSA following delivery.

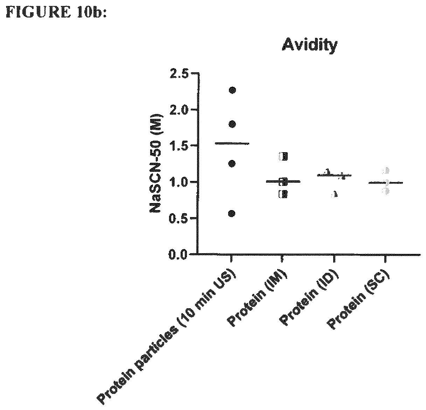

Results are depicted in Figure 10a. Immune response to BSA in the transdermal group (Group 1) was comparable to that of the injection groups, showing successful delivery of BSA via the transdermal route and successful generation of immune response to the administered protein. Figure 10(b) also shows that the transdermal group generated particles with the highest avidity.

### Transdermal delivery of DNA encoding luciferase via cavitation of protein particles

Particles according to the invention, or polymer particles produced in accordance with WO 2015/075422, were applied to a mouse flank. 8 mice were used in the experiment, and each mouse received two separate particle preparations, one applied to each flank. Thus, 16 experiments were carried out in total as follows:
- 4 x protein particles + DNA Luc (10 minute US exposure)
- 4 x protein particles + DNA Luc (2 minute US exposure)
- 4 x polymer particles + DNA Luc (10 minute US exposure)
- 4 x protein particles + DNA luc (10 minute No US Sham)

Protein particles used were particles produced in accordance with Example 1 above. Polymer particles were produced in accordance with WO 2015/075422. All samples were provided in PBS. Protein particles were provided as 9mg protein in 1.8ml PBS. All samples contained 50µg of DNA encoding luciferase. The particles/DNA were administered to each mouse by direct application to the mouse flank (with or without application of ultrasound). Ultrasound (US) was applied using the method described in *"In Vivo Cavitation Ultrasound Methodology"* above, for the indicated exposure time.

Successful transdermal delivery was assessed by the presence of luciferase following administration. Figure 11 shows bio-luminescence measurements following each mode of administration, and compares this with bio-luminscence generated following direct injection (via intramuscular, intradermal or subcutaneous injection) of DNA encoding luciferase. Injected solutions were identical to the protein particle solutions used for transdermal administration.

Transdermal administration of DNA using a composition comprising the DNA together with the particles of the invention produced comparable or significantly greater luminescence in all cases when compared to current gold standard cavitation agent (polymer particles).

Particles according to Example 18, together with luciferase pDNA, were also administered transdermally by the same method. Figure 8A shows the energy detected by PCD over time at 1.7 MPa during in vivo transdermal delivery of luciferase pDNA. At three different timepoints the frequency domain is plotted to show that inertial cavitation was dominant. A 2 MHz high-pass filter was used in acquiring the acoustic emissions data from the passive cavitation detector, hence no signal was visible below 2MHz on the spectrograms.

### Transdermal Vaccine

The aim of this experiment was to establish that inventive particles comprising an antigen protein (Covid19 spike protein) and containing the adjuvent imiquimod inside the oil core could enhance the immune response and compare with that of non imiquimod particles, native protein or native protein co-injected with imiquimod.

Mice were treated by administration of a composition as set out below. Each mouse received two separate particle preparations, one applied to each flank. The preparations applied were as follows. All preparations were made up in PBS:
- PBS intradermal (ID) injection
- Covid19 protein (5ug) ID injection
- Covid19 protein (5ug) + imiquimod (0.1ug) ID injection
- Covid19 protein particles (Ex 16) (providing 5ug protein) ID injection
- Covid19 protein particles with imiquimod core (Ex 16A) (providing 5ug protein and 0.1ug imiquimod) ID injection
- Covid 19 protein particles with imiquimod core (Ex 16A) (providing 5ug protein and 0.1ug imiquimod) transdermal + 10 minute US exposure

Covid spike protein used was SARS-CoV-2, S1, RBD, Cambridge Bioscience, 230-30162-1000. Protein was either used in native form, or prepared into particles produced in accordance with Example 16 or 16A above. The compositions set out above were administered to each mouse either by direct application to the mouse flank, or by intradermal injection as indicated above. The total protein content of each component administered was 5ug. It is important to note that for injection, the full 5ug is delivered to the mouse, whereas for transdermal administration, only a fraction of the 5ug protein is delivered. Total imiquimod content of samples, where relevant, was 0.1ug. The same amount of imiquimod is present whether the imiquimod is included in the core of the particle or provided separately. Where ultrasound was used, this was carried out as discussed in *"In Vivo Cavitation Ultrasound Methodology"* above.

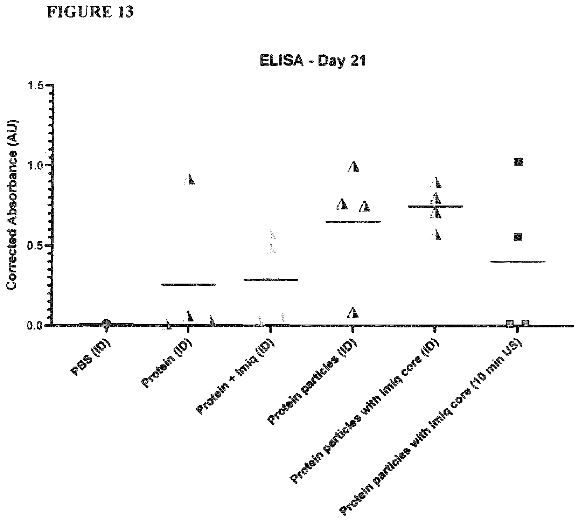

Mouse serum was sampled at intervals after administration of protein. An ELISA assay specific to covid spike protein RBD domain was used to assess generation of antibodies. Figure 13 shows the results of a day-21 ELISA assay. Protein particles with an imiquimod core generated higher levels of antibodies than native spike proteins delivered intradermally. Given the lower amount of protein which is expected to be delivered via this route, the greater level of immune response is surprising.

## Claims

1. A transdermal vaccine composition comprising a plurality of polypeptide particles and at least one pharmaceutically acceptable carrier or diluent, wherein the plurality of polypeptide particles are suitable for inducing cavitation on exposure to ultrasound, and wherein each particle comprises:
- a polypeptide shell; and optionally
- a core comprising a water immiscible liquid and/or one or more adjuvants;
wherein the particle has one or more surface indentations.

2. A transdermal vaccine composition according to claim 1, wherein the polypeptide comprises a pathogenic antigen protein and/or wherein the composition further comprises a vaccine, preferably a DNA or RNA vaccine.

3. A transdermal vaccine composition according to claim 1 or claim 2, wherein the core comprises one or more adjuvants, wherein the one or more adjuvants are preferably selected from imiquimod and squalene.

4. A transdermal vaccine composition according to any one of the preceding claims, wherein the polypeptide comprises a pathogenic antigen protein, preferably wherein the pathogenic antigen protein is selected from, a covid spike protein, HepB surface antigen protein, hemagglutinin, an influenza neuraminidase, filamentous hemagglutinin, pneumococcal surface protein A, Neisserial adhesin A, Neisserial Heparin Binding Antigen, factor H binding protein, and HPV-16 E6 or E7 fusion protein.

5. A transdermal vaccine composition according to any one of claims 1 to 4, wherein the polypeptide comprises an adjuvant polypeptide, preferably wherein the adjuvant polypeptide is one or more polypeptides selected from non-human albumin proteins, sFLT ligand, cytokines and chemokines, macrophage colony stimulating factor (M-CSF), tumour necrosis factor (TNF), and granulocyte and macrophage colony stimulating factor (GM-CSF).

6. A transdermal vaccine composition according to any one of claims 1 to 5, wherein the composition further comprises a DNA or RNA vaccine.

7. A transdermal vaccine composition according to any one of the preceding claims, wherein the particle size is in the range of from 100nm to 10000nm, preferably from 200nm to 1000nm.

8. A transdermal vaccine composition according to any one of the preceding claims, wherein the cross section of at least one surface indentation forms a conic section; and/or wherein at least one surface indentation has a depth of at least 10nm; and/or wherein the particle comprises one or two indentations having an opening size which is 20% or more of the size of the particle.

9. A transdermal vaccine composition according to any one of the preceding claims, wherein the polypeptide particles are capable of generating inertial cavitation when the composition is exposed to ultrasound at 1.8MPa and 265kHz.

10. A polypeptide particle for inducing cavitation on exposure to ultrasound, the particle comprising:
- a polypeptide shell comprising a pathogenic antigen protein as defined in claim 4, and/or an adjuvant polypeptide as defined in claim 5; and optionally
- a core comprising a water immiscible liquid and/or one or more adjuvants, the adjuvants being as defined in claim 3;
wherein the particle has one or more surface indentations.

11. A polypeptide particle for inducing cavitation on exposure to ultrasound, the particle comprising:
- a polypeptide shell wherein the polypeptide shell optionally comprises a pathogenic antigen protein as defined in claim 4; and
- a core comprising one or more adjuvants and optionally a water-immiscible liquid, wherein the adjuvants are as defined in claim 3;
wherein the particle has one or more surface indentations.

12. A pharmaceutical adjuvant composition comprising a plurality of polypeptide particles according to claim 11 together with one or more pharmaceutically acceptable carriers or diluents.

13. A method of producing a polypeptide particle according to claim 10 or 11, comprising:
- providing a water-immiscible phase comprising one or more volatile components, optionally one or more non-volatile components, and optionally one or more adjuvants as defined in claim 3, optionally wherein the ratio of volatile component to non-volatile component in the water-immiscible phase is from 1:20 to 20:1;
- mixing said water-immiscible phase with an aqueous solution of at least one polypeptide, wherein the at least one polypeptide optionally comprises a pathogenic antigen protein as defined in claim 4 and/or an adjuvant polypeptide as defined in claim 5;
- cross-linking the polypeptide to generate a particle having a core comprising the water-immiscible phase and a shell comprising at least one polypeptide; and
- creating one or more indentations in the particle by subjecting the particle to reduced pressure conditions, to remove volatile component from the core; and optionally
- purifying the particle.

14. A method according to claim 13, which further comprises:
- drying the particle; and
- optionally re-suspending the particle in a liquid medium; and wherein the method may also further comprise:
- lyophilising the particle; and
- optionally re-suspending the particle in a liquid medium;
wherein preferably the lyophilisation and optional resuspension are carried out after drying the particle and re-suspending the particle in a liquid medium.

15. A polypeptide particle or composition according to any one of claims 1 to 12, for use in a method of vaccination, (a) wherein the method preferably comprises applying the polypeptide particle or composition to the skin, applying ultrasound and inducing inertial cavitation, such that the polypeptide particle or composition is delivered transdermally, wherein ultrasound is preferably delivered at a frequency of from 100 to 500 kHz and a peak negative pressure of from 0.5 MPa to 2.0 MPa; and/or (b) wherein the polypeptide particle or transdermal vaccine composition is preferably administered together with a DNA or RNA vaccine.
